# EUROPEAN PATENT SPECIFICATION

(11) **EP 1 444 267 B1**
(45) Date of publication and mention of the grant of the patent: **10.08.2016**
(21) Application number: 02770573.0
(22) Date of filing: 15.10.2002
(51) Int. Cl.: C07K 16/44, A61K 39/00, A61K 45/06, C07K 16/30

(54) **AFFINITY ENHANCEMENT AGENTS**
AFFINITÄTSVERSTÄRKENDE MITTEL
AGENTS D'AUGMENTATION DE L'AFFINITE

(30) Priority: 15.10.2001 US 328835 P; 21.12.2001 US 341881 P
(43) Date of publication of application: 11.08.2004
(73) Proprietor: Immunomedics, Inc., Morris Plains, NJ 07950 (US); IBC Pharmaceuticals, Morris Plains, NJ 07950 (US)
(72) Inventor: CHANG, Chien-Hsing Ken, Morris Plains, NJ 07945 (US); ROSSI, Edmund, Morris Plains, NJ 07945 (US)
(74) Representative: Bohmann, Armin K.
(86) International application number: PCT/US2002/032717
(87) International publication number: WO 2003/033653

(56) References cited:
- WO-A-99/66951
- WO-A-03/057829
- US-A- 5 256 395
- US-A- 5 837 242
- HILLAIRET DE BOISFERON ET AL.: 'Enhanced targeting specificity to tumor cells by simultaneous recognition of two antigens' BIOCONJUGATE CHEM. vol. 11, 2000, pages 452 - 460, XP002967821
- JANEVIK-IVANOVSKA E ET AL: "BIVALENT HAPTEN-BEARING PEPTIDES DESIGNED FOR IODINE-131 PRETARGETED RADIOIMMUNOTHERAPY", BIOCONJUGATE CHEMISTRY, ACS, WASHINGTON, DC, US, vol. 8, no. 4, 1 January 1997 (1997-01-01) , pages 526-533, XP000929131, ISSN: 1043-1802, DOI: 10.1021/BC970083H
- MOREL A ET AL: "Recognition of imidazole and histamine derivatives by monoclonal antibodies", MOLECULAR IMMUNOLOGY, PERGAMON, GB, vol. 27, no. 10, 1 October 1990 (1990-10-01), pages 995-1000, XP024781220, ISSN: 0161-5890, DOI: 10.1016/0161-5890(90)90122-G [retrieved on 1990-10-01]

## Description

### FIELD OF THE INVENTION

The present invention is related to a multivalent, multi-specific binding protein comprising a histamine-succinyl-glycyl (HSG) binding site having an affinity towards molecules containing a HSG moiety and a carcinoembryonic antigen (CEA) binding site having an affinity towards CEA, wherein said HSG antigen binding site comprises a first and second polypeptide segments that must associate with each other to form said HSG antigen binding site; a host cell comprising a plasmid encoding the multivalent, multi-specific binding protein; a method of producing a binding protein, wherein the method comprises culturing the host cell; the multivalent, multi-specific binding protein for use in the treatment of a disease; the multivalent, multi-specific binding protein for use in diagnosis of a disease; and the use of the multivalent, multi-specific binding protein in the preparation of a medicament for detecting or treating a human disorder.

### BACKGROUND OF THE INVENTION

Man-made binding proteins, in particular monoclonal antibodies and engineered antibodies or antibody fragments, have been tested widely and shown to be of value in detection and treatment of various human disorders, including cancers, autoimmune diseases, infectious diseases, inflammatory diseases, and cardiovascular diseases [Filpula and McGuire, Exp. Opin. Ther. Patents (1999) 9: 231-245]. For example, antibodies labeled with radioactive isotopes have been tested to visualize tumors after injection to a patient using detectors available in the art. The clinical utility of an antibody or an antibody-derived agent is primarily dependent on its ability to bind to a specific targeted antigen. Selectivity is essential for delivering a diagnostic or therapeutic agent, such as isotopes, drugs, enzymes, toxins, cytokines, hormones, growth factors, or conjugated derivatives thereof, to a target location during the detection and treatment phases of a human disorder, particularly if the diagnostic or therapeutic agent is toxic to normal tissue in the body.

The major limitations of antibody systems are discussed in Goldenberg, The American Journal of Medicine (1993) 94: 298-299. The essential parameters in the detection and treatment techniques are the amount of the injected dose specifically localized at the site(s) where target cells are present and the uptake ratio, *i.e.,* the ratio of the concentration of specifically bound antibody to that of the radioactivity present in surrounding normal tissues. When an antibody is injected into the blood stream, it passes through a number of compartments as it is metabolized and excreted. The antibody must be able to locate and bind to the target cell antigen while passing through the rest of the body. Factors that control antigen targeting include antigen location, antigen density, antigen accessibility, cellular composition of pathologic tissue, and the pharmokinetics of the targeting antibodies. Other factors that specifically affect tumor targeting by antibodies include expression of the target antigens, both in tumor and other tissues, and bone marrow toxicity resulting from the slow blood-clearance of the radiolabeled antibodies.

The amount of targeting antibodies accreted by the targeted tumor cells is influenced by the vascularization and barriers to antibody penetration of tumors, as well as intratumoral pressure. Non-specific uptake by non-target organs such as the liver, kidneys or bone-marrow is another major limitation of the technique, especially for radioimmunotherapy, where irradiation of the bone marrow often causes the dose-limiting toxicity.

One suggested solution, referred to as the "Affinity Enhancement System" (AES), is a technique especially designed to overcome the deficiencies of tumor targeting by antibodies carrying diagnostic or therapeutic radioisotopes [U.S. 5,256,395 (1993), Barbet et al., Cancer Biotherapy & Radiopharmaceuticals (1999) 14: 153-166]. The AES requires a radiolabeled bivalent hapten and an anti-tumor/anti-hapten bispecific antibody that recognizes both the target tumor and the radioactive hapten. The technique involves injecting the bispecific antibody into the patient and allowing the bispecific antibody to localize at the target tumor. After a sufficient amount of time for the unbound antibody to clear from the blood stream, the radiolabeled hapten is administered. The hapten binds to the antibody-antigen complex located at the site of the target cell to obtain diagnostic or therapeutic benefits. The unbound hapten clears the body. Barbet mentions the possibility that a bivalent hapten may crosslink with a bispecific antibody, when the latter is bound to the tumor surface. As a result, the radiolabeled complex is more stable and stays at the tumor for a longer period of time.

Bispecific antibodies prepared by chemically crosslinking two different Fab' fragments have been employed successfully, along with applicable bivalent haptens, to validate the utility of the AES for improved tumor targeting both in animal models and in human patients. However, there remains a need in the art for production of bispecific antibodies by recombinant DNA technology to assess whether such engineered antibodies have merits for the AES. Specifically, there remains a need for an antibody-based agent that exhibits enhanced uptake at targeted antigens, fast clearance from the blood, and optimal protection of normal tissues and cells from toxic pharmaceuticals.

International patent application WO 03/057829 is related to methods of generating multispecific, multivalent agents from V_{H} and V_{L} domains.

International patent application 99/66951 is related to the use of bi-specific antibodies for pre-targeting diagnosis and therapy.

US patent US 5,256,395 is related to affinity enhancement immunological reagents for *in vivo* detection and killing of specific target cells.

De Boisferon H et al. (Bioconjugate Chem, vol. 11, 2000, pp. 452-460) report on enhanced targeting specificity to tumor cells by simultaneous recognition of two antigens.

US patent US 5,837,242 is related to multivalent and multispecific binding proteins, their manufacture and use.

Janevik-Ivanovska E et al. (Bioconjugate Chemistry, vol. 8, no. 4, 1997, pp. 526-533) report on bivalent hapten-bearing peptides designed for Iodine-131 pretargeted radioimmunotherapy.

Morel A et al. (Molecular Immunology, vol. 27, no. 10, 1990, pp. 995-1000) report on recognition of imidazole and histamine derivatives by monoclonal antibodies.

### SUMMARY OF THE INVENTION

The problem underlying the present invention is solved by the subject matter of the attached independent claims. Preferred embodiments may be taken from the attached dependent claims.

More specifically, the problem underlying the present invention is solved in a first aspect by a multivalent, multi-specific binding protein comprising a histamine-succinyl-glycyl (HSG) binding site having an affinity towards molecules containing a HSG moiety and a carcinoembryonic antigen (CEA) binding site having an affinity towards CEA, wherein said HSG antigen binding site comprises a first and second polypeptide segments that must associate with each other to form said HSG antigen binding site, wherein
said first polypeptide segment comprises a heavy chain variable region comprising an amino acid sequence selected from the group consisting of 
EVILVESGGDLVKPGGSLKLSCAASGFTFSIYTMSWLRQTPGKGLEWVATLSGDGDD
IYYPDSVKGRFTISRDNAKNSLYLQMNSLRAEDTALYYCARVRLGDWDFDVWGQGT
TVSVSS (VH 679) and
 
EVQLVESGGDLVKPGGSLKLSCAASGFTFSIYTMSWLRQTPGKGLEWVATLSGDGD
DIYYPDSVKGRFTISRDNAKNSLYLQMNSLRAEDTALYYCARVRLGDWDFDVWGQ
GTTVSVSS (VH 679 I3Q),
 and said second polypeptide segment comprises a light chain variable region comprising an amino acid sequence selected from the group consisting of

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLCTFGAGTKLELKR
(VK 679) and

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLSTFGAGTKLELKR
(VK 679 C101S).

In an embodiment of the first aspect said first polypeptide segment comprises a heavy chain variable region comprising an amino acid sequence of 
EVILVESGGDLVKPGGSLKLSCAASGFTFSIYTMSWLRQTPGKGLEWVATLSGDGDD
IYYPDSVKGRFTISRDNAKNSLYLQMNSLRAEDTALYYCARVRLGDWDFDVWGQGT
TVSVSS (VH 679 I3Q),
 and said second polypeptide segment comprises a light chain variable region comprising an amino acid sequence of

IVMTQSPSSLAVSPGERVTLTCKSSQSLFNSRTRKNYLGWYQQKPGQSPKLLIYWAST
RESGVPDRFSGSGSGTDFTLTINSLQAEDVAVYYCTQVYYLCTFGAGTKLELKR (VK
679).

In an embodiment of the first aspect said first polypeptide segment comprises a heavy chain variable region comprising an amino acid sequence of 
EVQLVESGGDLVKPGGSLKLSCAASGFTFSIYTMSWLRQTPGKGLEWVATLSGDGD
DIYYPDSVKGRFTISRDNAKNSLYLQMNSLRAEDTALYYCARVRLGDWDFDVWGQ
GTTVSVSS (VH 679 I3Q),
 and said second polypeptide segment comprises a light chain variable region comprising an amino acid sequence of

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLSTFGAGTKLELKR
(VK 679 C101S).

In an embodiment of the first aspect said CEA antigen binding site comprises a third and fourth polypeptide segments that must associate with each other to form said CEA antigen binding site.

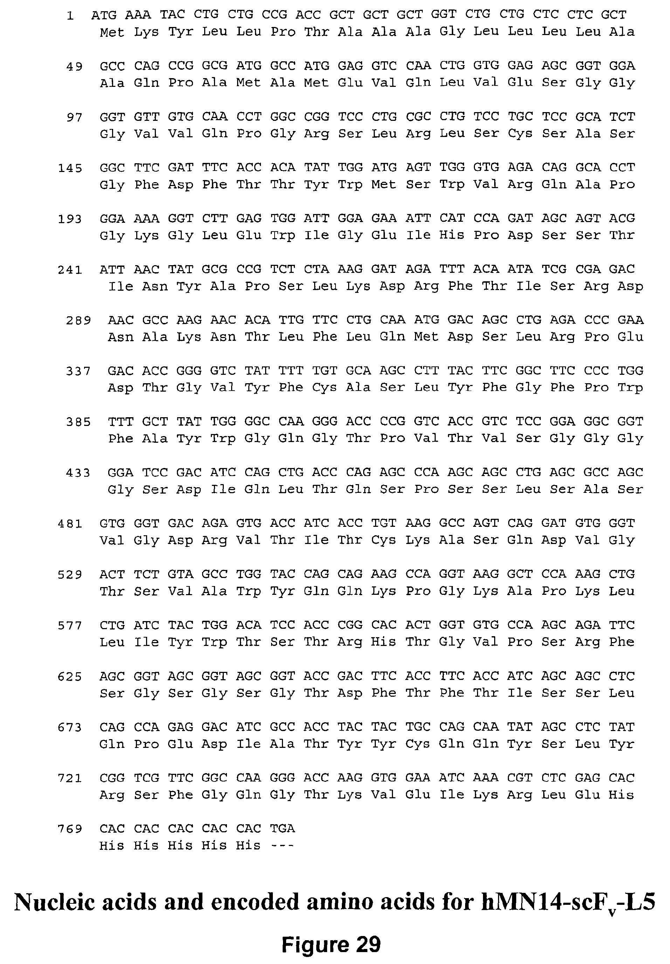

In an embodiment of the first aspect said third polypeptide segment comprises a VH polypeptide of hMN14 MAb of Fig. 29, and said fourth polypeptide segment comprises a VK polypeptide of hMN14 MAb of Fig. 29.

In an embodiment of the first aspect said first and third polypeptide segments are connected by a first linker and said second and fourth polypeptide segments are connected by a second linker.

In an embodiment of the first aspect said first linker and said second linker each comprise five or less amino acid residues.

In an embodiment of the first aspect said binding protein is a diabody.

In an embodiment of the first aspect said diabody is selected from the group consisting of a diabody comprising amino acid sequences shown in Fig. 30 and Fig. 31, a diabody comprising amino acid sequences shown in Fig. 32 and Fig. 33, a diabody comprising amino acid sequences shown in Fig. 34 and Fig. 35, and a diabody comprising amino acid sequences shown in Fig. 37 and 38.

In an embodiment of the first aspect said first, second, third and fourth polypeptide segments are each encoded by a first, second, third and fourth cDNA, respectively.

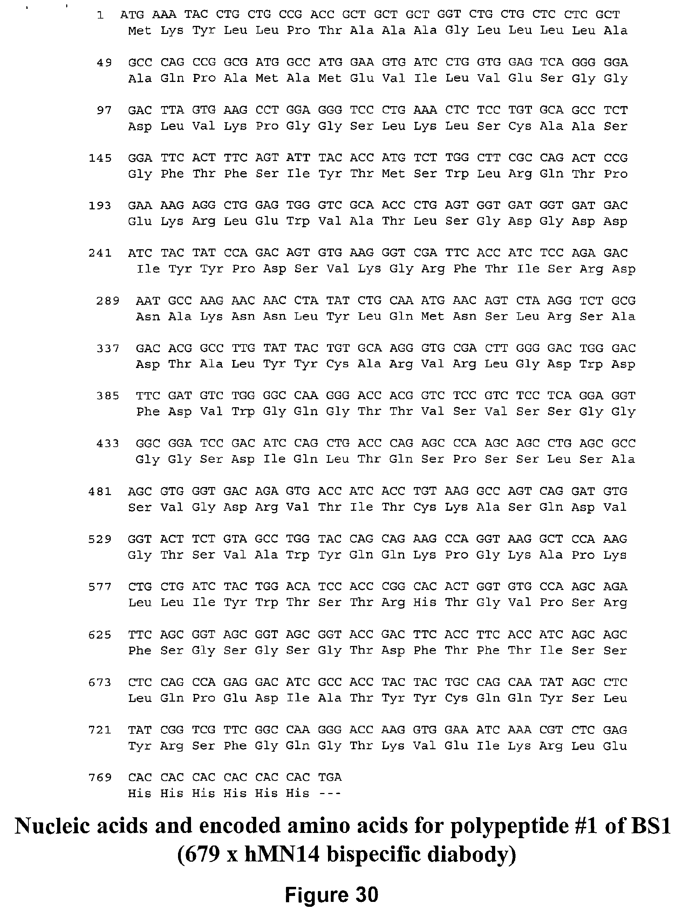

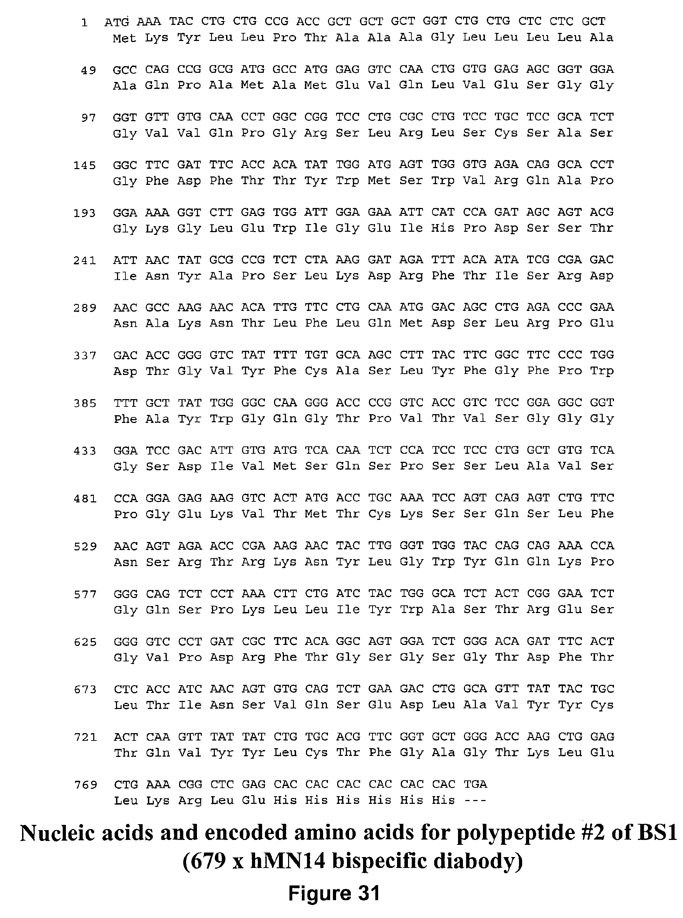

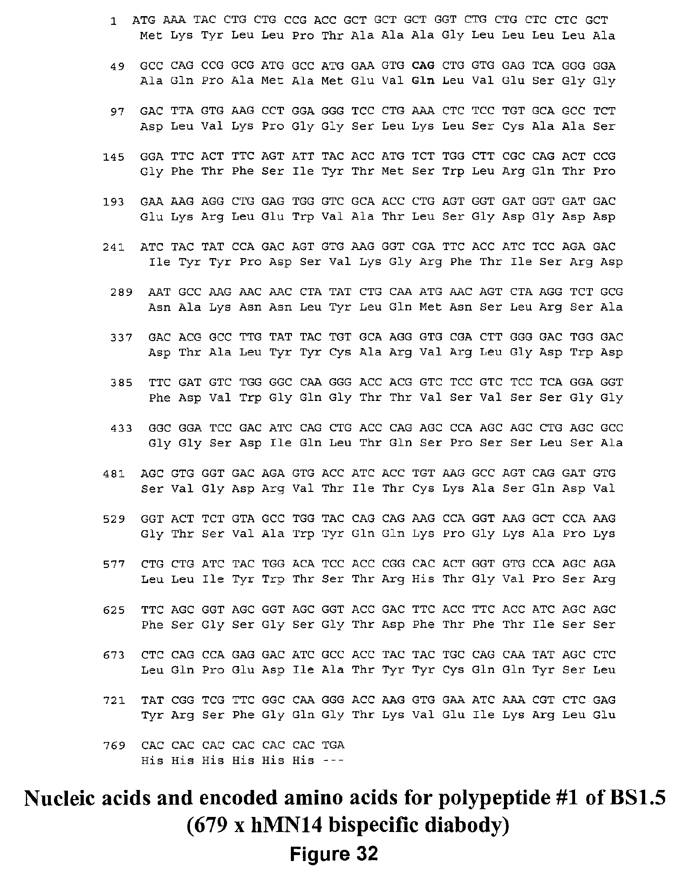

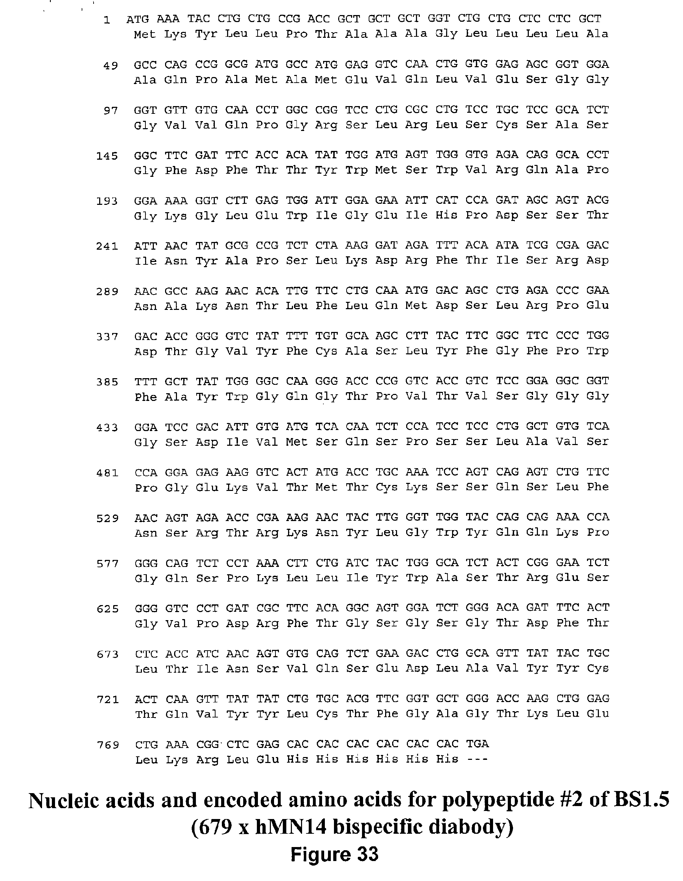

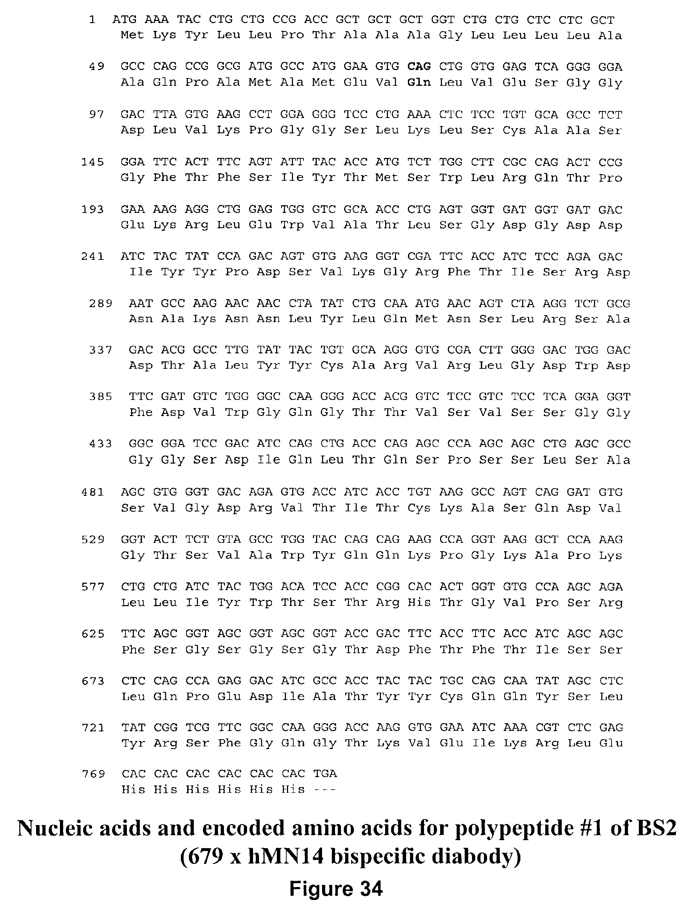

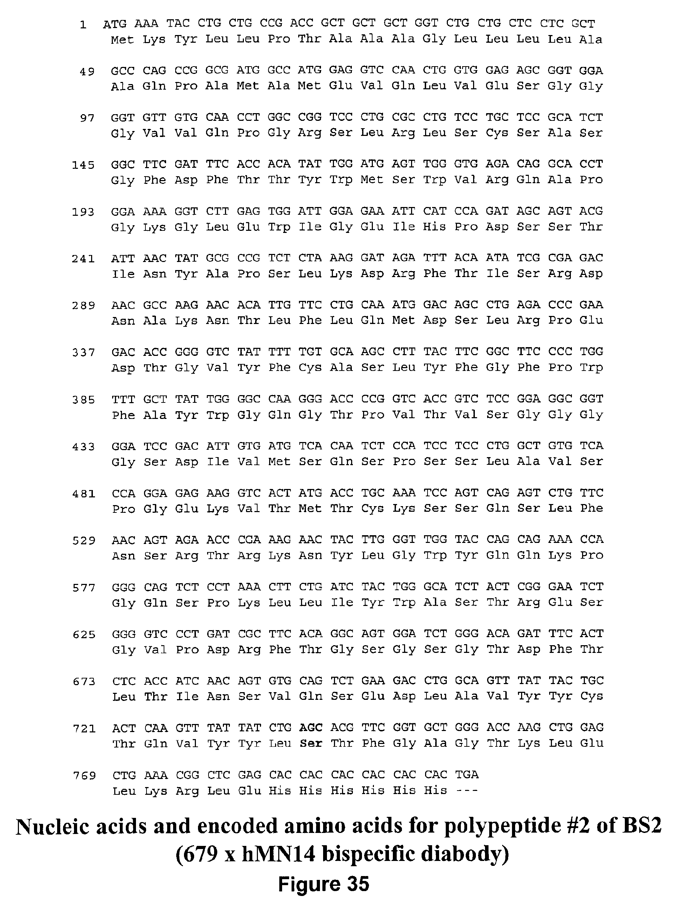

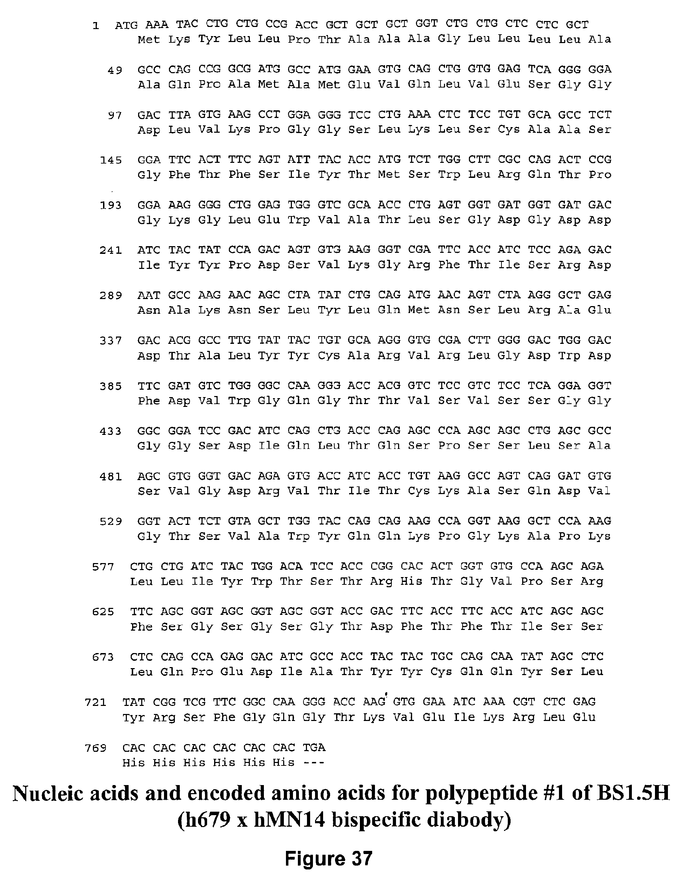

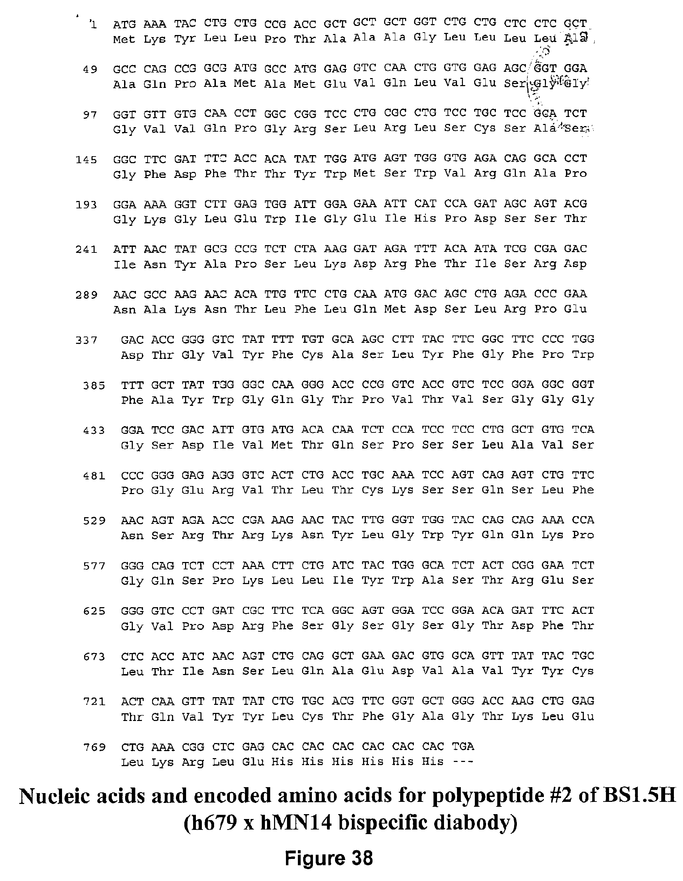

In an embodiment of the first aspect said first cDNA comprises nucleotide sequences shown in Fig. 30 and Fig. 31, wherein said second cDNA comprises nucleotide sequences shown in Fig. 32 and Fig. 33, wherein said third cDNA comprises nucleotide sequences shown in Fig. 34 and 35, and wherein said fourth cDNA comprises nucleotide sequences shown in Fig. 37 and 38.

In an embodiment of the first aspect said first and third cDNAs are contained on a single nucleic acid molecule, or wherein said second and fourth cDNAs are contained on a single nucleic acid molecule, or wherein said first, second, third and fourth cDNAs are on a single expression cassette, wherein preferably said expression cassette is contained in a plasmid.

More specifically, the problem underlying the present invention is solved in a second aspect by a host cell comprising a plasmid as defined in the above embodiment of the first aspect.

More specifically, the problem underlying the present invention is solved in a third aspect by a method of producing a binding protein, comprising culturing the host cell of the second aspect in a suitable medium, and separating said binding protein from said media.

More specifically, the problem underlying the present invention is solved in a fourth aspect by a binding protein of the first aspect for use in treatment of a disease, wherein the treatment comprises delivering a therapeutic agent, or a combination of the therapeutic agent and a diagnostic agent to a target, comprising:
a) administering the binding protein to a subject;
b) waiting a sufficient amount of time for an amount of the non-bound binding protein to clear the subject's blood stream; and
c) administering to said subject a carrier molecule comprising the therapeutic agent or a combination of the therapeutic agent and a diagnostic agent, that binds to a binding site of the binding protein.

More specifically, the problem underlying the present invention is solved in a fifth aspect by a binding protein of the first aspect for use in diagnosis of disease, wherein the diagnosis comprises delivering a diagnostic agent or a combination of the diagnostic agent and a therapeutic agent to a target, comprising:
a) administering the binding protein to a subject;
b) waiting a sufficient amount of time for an amount of the non-bound binding protein to clear the subject's blood stream; and
c) administering to said subject a carrier molecule comprising the diagnostic agent or a combination of the diagnostic agent and a therapeutic agent, that binds to a binding site of the binding protein.

In an embodiment of the fourth and fifth aspect said carrier molecule binds to more than one binding site of the binding protein.

In an embodiment of the fourth aspect said therapeutic agent is selected from the group comprising, drugs, toxins, cytokines, hormones, growth factors, conjugates, and radionuclides.

More specifically, the problem underlying the present invention is solved in a sixth aspect by the use of a binding protein of the first aspect in the preparation of a medicament for detecting or treating a human disorder, wherein the binding protein of the first aspect is administered to a subject; a sufficient amount of time is waited for an amount of the non-bound binding protein to clear the subject's blood stream; and a carrier molecule comprising a diagnostic agent, a therapeutic agent, or a combination thereof, that binds to a binding site of the binding protein is administered to the subject.

In an embodiment of the sixth aspect said human disorder is selected from the group comprising cancer, autoimmune diseases, infectious diseases, cardiovascular diseases, and inflammatory diseases.

### BRIEF DESCRIPTION OF THE DRAWINGS

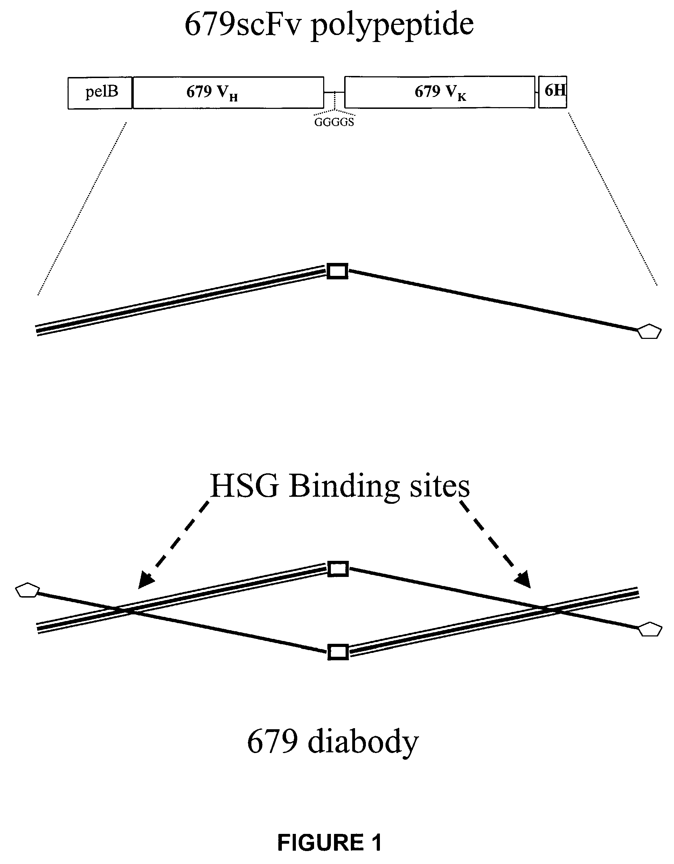

*Figure 1* shows a schematic representation of the 679 single chain Fv (scFv) polypeptide that is synthesized in *E. coli* from the 679-scFv-L5 expression plasmid and forms a 679 diabody. The gene construct for the un-processed polypeptide contains the pelB signal peptide, 679V_{H} and V_{K} coding sequences coupled by a 5 amino acid linker, Gly-Gly-Gly-Gly-Ser (G₄S), and the carboxyl terminal six histidine (His) affinity tag. The figure also shows a stick figure drawing of the mature polypeptide after proteolytic removal of the pelB leader peptide and a stick figure drawing of a 679 diabody, including the HSG binding sites.

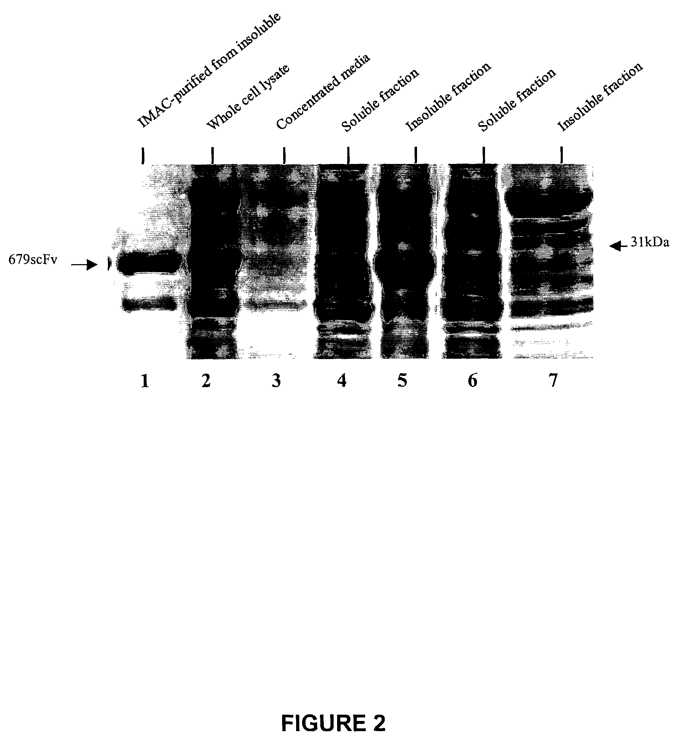

*Figure 2* shows a SDS-PAGE gel stained with Coomassie blue that is used to analyze the expression of 679 scFv from 679scFv-L5-transformed *E. coli* BL21 p-LysS cultures: lanes 1- 5, induced with isopropyl-β-D-galactopyranoside (IPTG) overnight at 20°C; lanes 6 and 7, not induced. In lane 3, the culture media was concentrated 10-fold. Soluble (lanes 4 and 6) and insoluble (lanes 5 and 7) proteins were fractionated by centrifugation of cell lysates (lane 2). 679scFv was purified from the insoluble fraction by Immobilized Metal Affinity Chromatography (IMAC) following solubilization in 8M urea (lane 1).

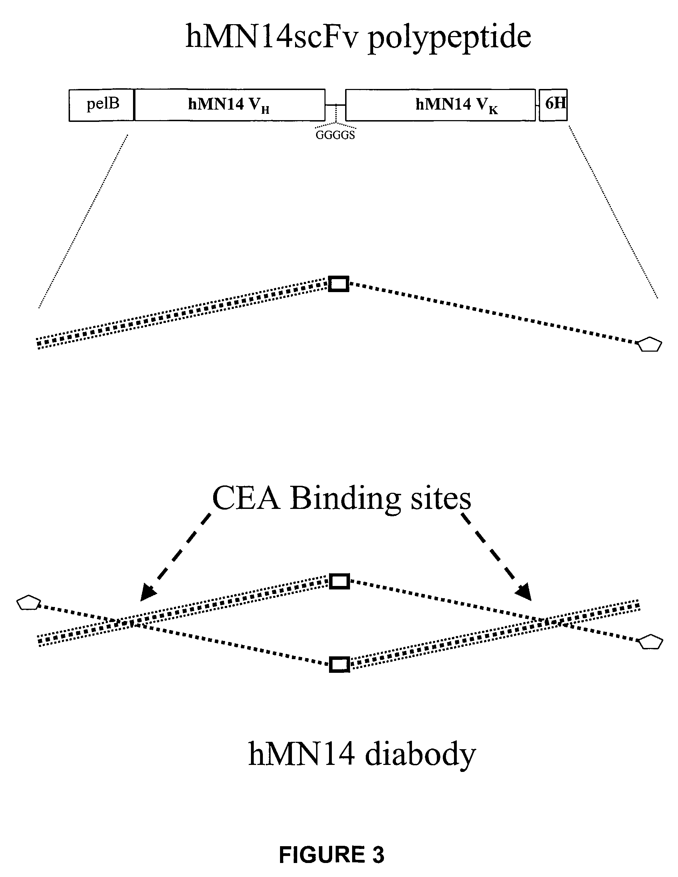

*Figure 3* shows a schematic representation of the hMN14scFv polypeptide that is synthesized in *E. coli* from the hMN14-scFv-L5 expression plasmid and forms a hMN14 diabody. The gene construct for the un-processed polypeptide contains the pelB signal peptide, hMN14V_{H} and V_{K} coding sequences coupled by a 5 amino acid linker, and the carboxyl terminal 6 histidine affinity tag. The figure also shows a stick figure drawing of the mature polypeptide following proteolytic removal of the pelB leader peptide, and a stick figure drawing of a hymn14 diabody, including CEA binding sites.

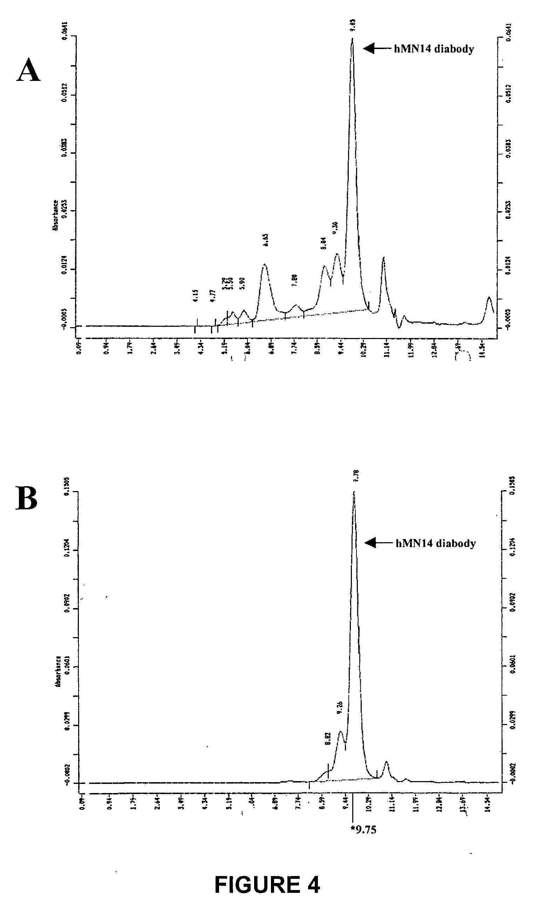

*Figure* 4 shows size-exclusion High Performance Liquid Chromatography (HPLC) analysis of purified hMN14 diabody. Figure A is the HPLC elution profile of IMAC-purified hMN14 diabody. The HPLC elution peaks of hMN14 diabody in figures A and B are identified with an arrow. Figure B is the HPLC elution profile of hMN14 diabody purified by WI2 anti-idiotype affinity chromatography. The *9.75 indicated on the x-axis of figure B is the HPLC retention time (9.75 min.) of control hMN14-Fab'-S-NEM (MW ~ 50 KDa).

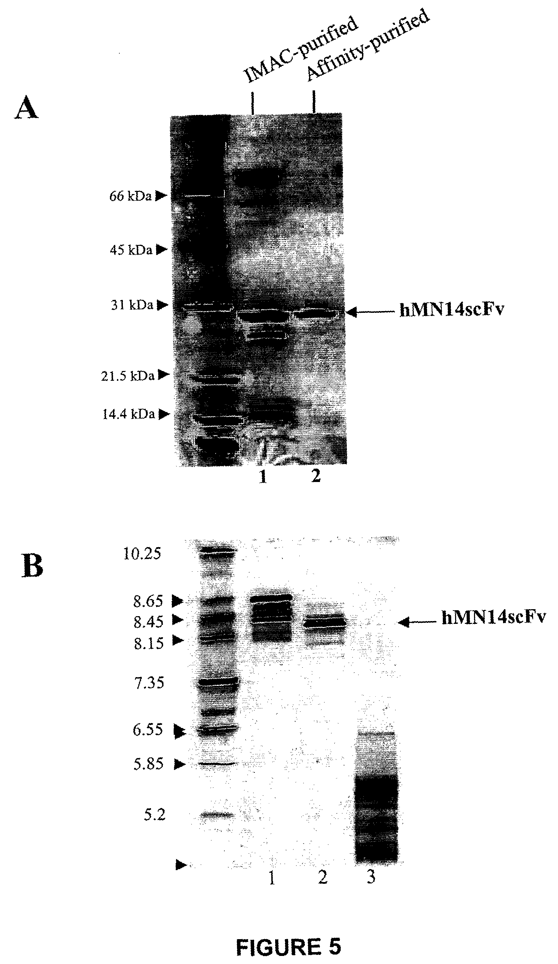

*Figure 5* shows reducing SDS-PAGE gel stained with Coomassie blue (figure A). The gel illustrates the purity of the hMN14 diabody samples following IMAC purification and WI2 anti-idiotype affinity chromatography. The positions of the Mᵣ standards and the hMN14scFv polypeptide are indicated with arrows. Lane 1 of figure A contains IMAC-purified hMN14 diabody. Lane 2 of the same figure contains affinity purified hMN14 diabody. Figure B is an isoelectric focusing (IEF) gel. The positions of pI standards and hMN14 diabody are indicated with arrows. Lane 1 of Figure B contains the hMN14 Fab'-S-NEM used as a standard. Lane 2 of the same figure contains the WI2 purified hMN14 diabody. Lane 3 contains the unbound flow through fraction from the WI2 affinity column and shows the proteins that are removed by this process.

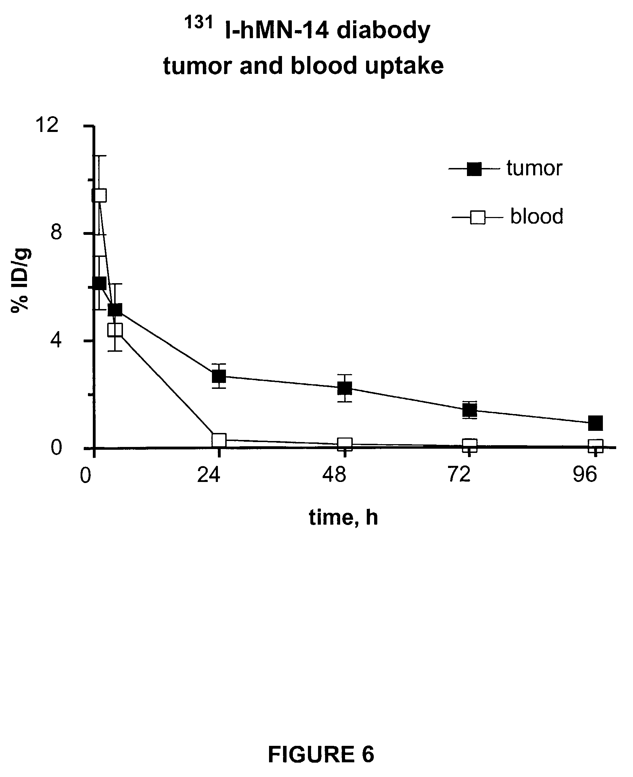

*Figure* 6 shows the levels of ¹³¹I-hMN14 diabody observed in a tumor and the blood over the first 96 hours after injection of the diabody. The concentration of ¹³¹I-hMN14 diabody, measured as the percentage of the injected dose per gram of tissue (%ID/g), is plotted vs. time. Solid squares mark the data points for tumor samples and open boxes mark those of blood samples.

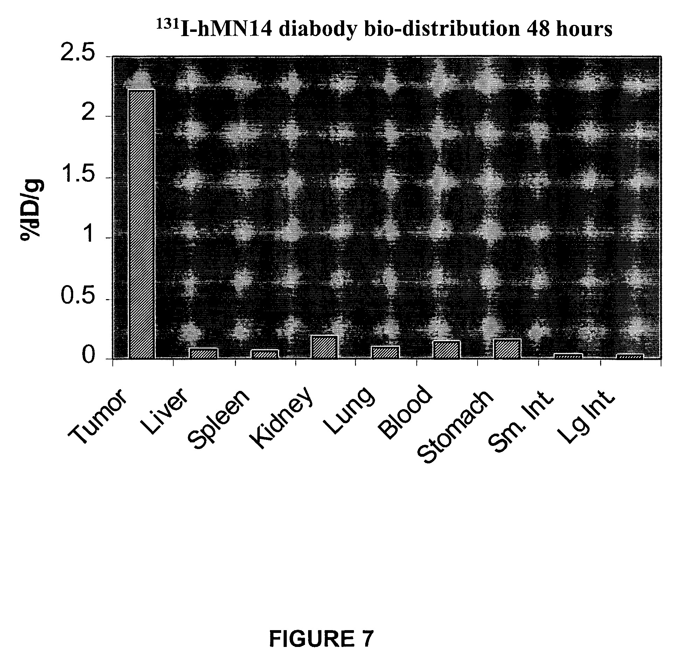

*Figure* 7 shows the biodistribution of ¹³¹I-hMN14 diabody 48 hours after injection in tumors and normal tissues, including liver, spleen, kidney, lungs, blood, stomach, small intestine, and large intestine. The concentration of ¹³¹I-hMN14 diabody is displayed as the percentage of the injected dose per gram of tissue (%ID/g).

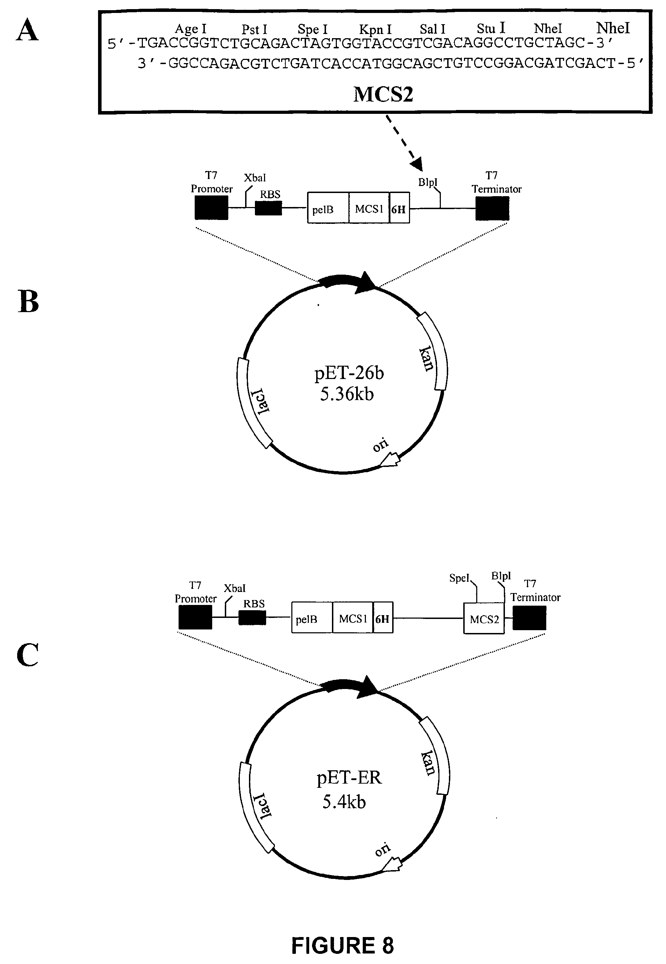

*Figure 8* shows a schematic representation of the creation of the pET-ER vector. Figure A illustrates the double stranded DNA sequence of MCS2. Restriction sites are indicated above the sequence. MCS2 was ligated into the BlpI restriction site of pET26b vector shown in Figure B. Figure C shows the diagram of pET-ER vector, including the MCS2 sequence.

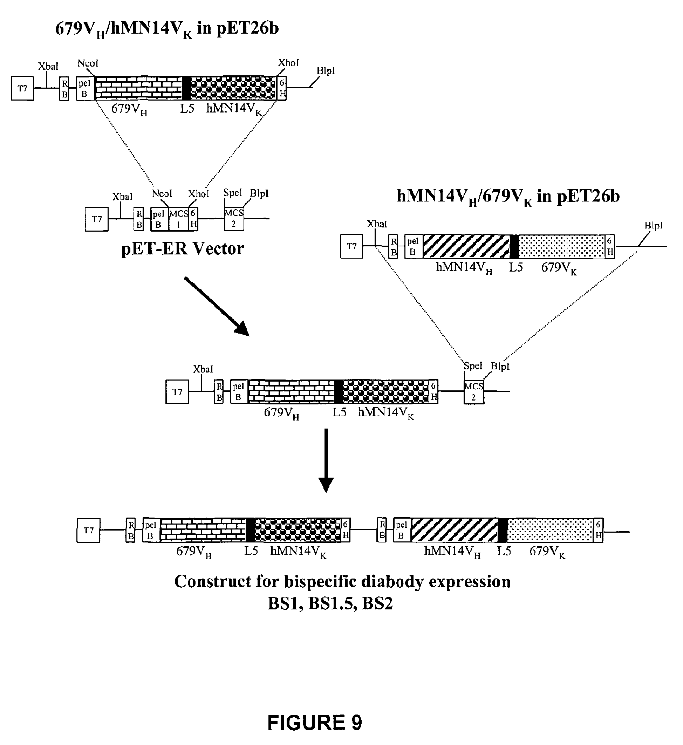

*Figure* 9 shows a schematic representation of the steps involved in the generation of constructs used for expression of three 679xhMN14 bispecific diabody variants represented by BS1, BS1.5 and BS2.

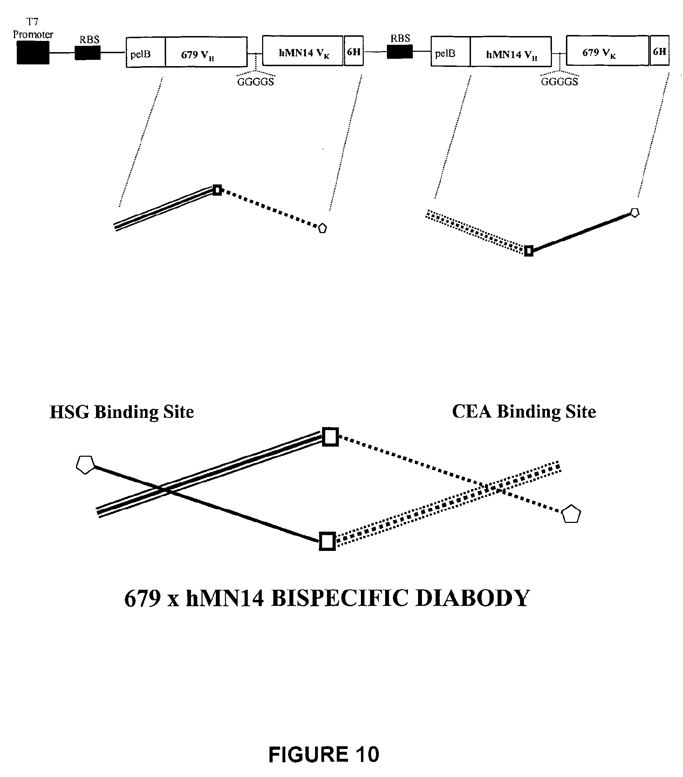

*Figure 10* shows a schematic representation of the di-cistronic expression cassette in the pET-ER vector and also stick figures of the two heterologous polypeptides as synthesized and the formation of 679 x hMN14 bispecific diabodies. The di-cistronic cassette codes for a single RNA message generated from T7 RNA polymerase via the T7 promoter. This message contains two ribosomal binding sites (RBS) and the coding sequences for the two heterologous polypeptides. Stick figure drawings show the two mature heterologous polypeptides, 679V_{H}(G₄S)hMN14V_{K}(Left) and hMN14V_{H}(G₄S)679V_{K} (Right) that are synthesized from the di-cistronic expression cassettes. The 679 x hMN14 bispecific diabody (BS1, BS1.5 or BS2) is represented as a stick figure drawing and is formed from the pairing of the heterologous polypeptides.

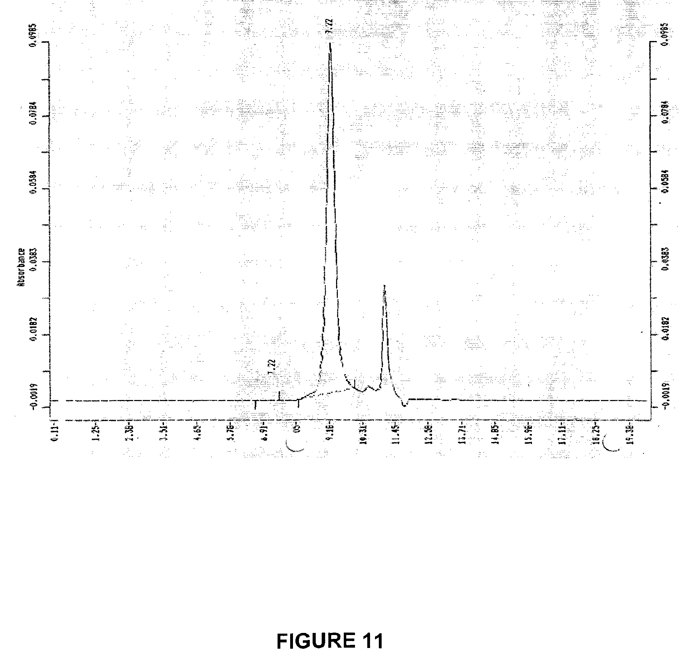

*Figure 11* shows a size-exclusion HPLC analysis of BS1.5 after purification. The HPLC elution peak of BS1.5 is at 9.22 min. Soluble proteins from an induced 5L culture were purified by Ni-NTA IMAC followed by Q-Sepharose anion exchange chromatography. The flow through fraction of the Q-Sepharose column was injected for HPLC analysis.

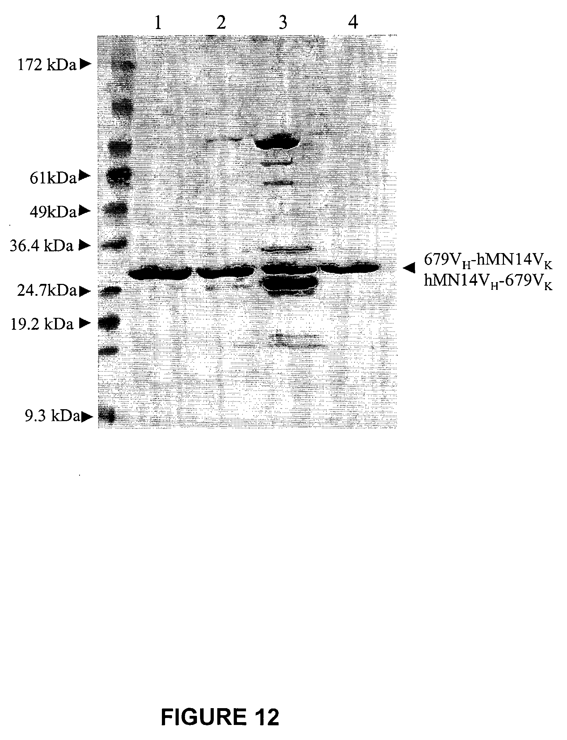

*Figure 12* shows a reducing SDS-PAGE gel stained with Coomassie blue and used to analyze the purification of BS2. Arrows indicate the positions of the Mᵣ standards and the BS2 polypeptide constituents, 679V_{H}-hMN14V_{K} and hMN14V_{H}-679V_{K}. Soluble proteins from an induced 5L culture were loaded on a 4 ml Ni-NTA column. The column was washed/eluted with a buffer containing 40mM imidazole (lane 3) and then eluted in two fractions with 100mM imidazole (lanes 1 and 2). Impurities in the 40mM imidazole eluate were removed by passing the eluate over a Q-Sepharose anion exchange column (lane 4).

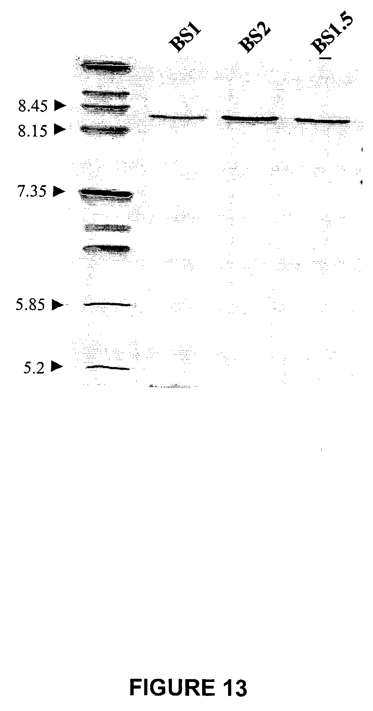

*Figure 13* shows the purity of BS 1, BS2 and BS1.5 through an IEF gel. These three diabodies were purified from soluble protein extracts by Ni-NTA IMAC followed by Q-Sepharose anion exchange chromatography. The positions of pI markers are indicated by arrows and the samples are identified above the lanes.

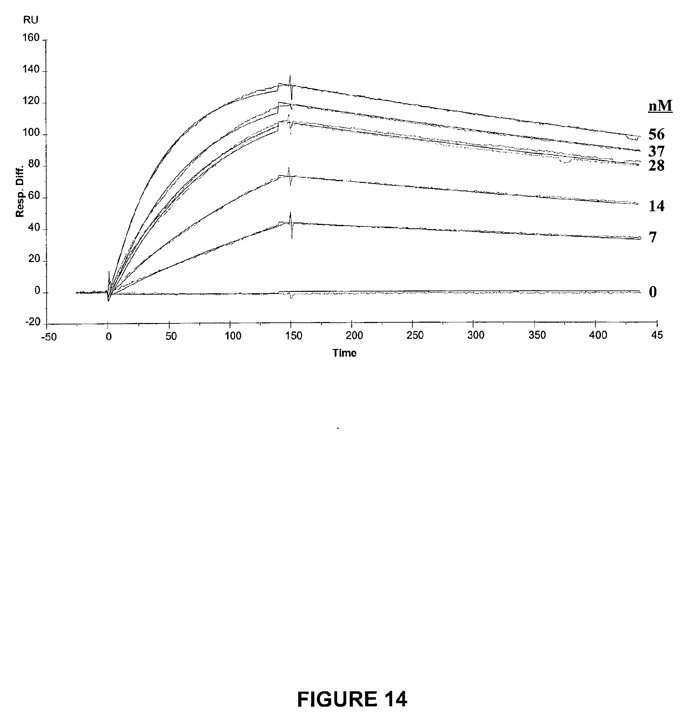

*Figure 14* shows BIAcore binding curves obtained for various concentrations of BS1.5 using a low-density HSG-coupled sensor chip. These data were used for calculation of the on-rates and off-rates.

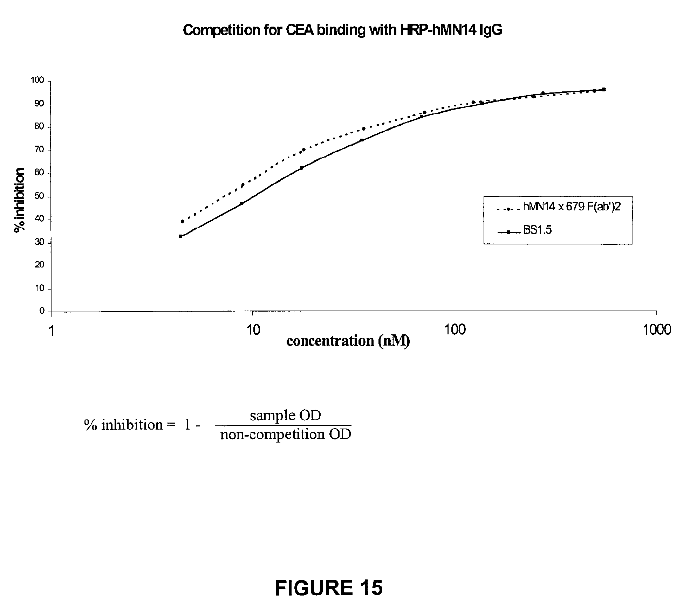

*Figure 15* is a graphical representation of the results of a competitive enzyme-linked immunosorbent assay (ELISA). HRP-conjugated hMN14 IgG (1 nM) was mixed with either BS1.5 or chemically linked 679 x hMN14 F (ab')₂ at concentrations ranging from 4 - 500 nM, prior to incubation in CEA-coated (0.5 µg/well) wells. The % inhibition is plotted vs. nM concentration of sample.

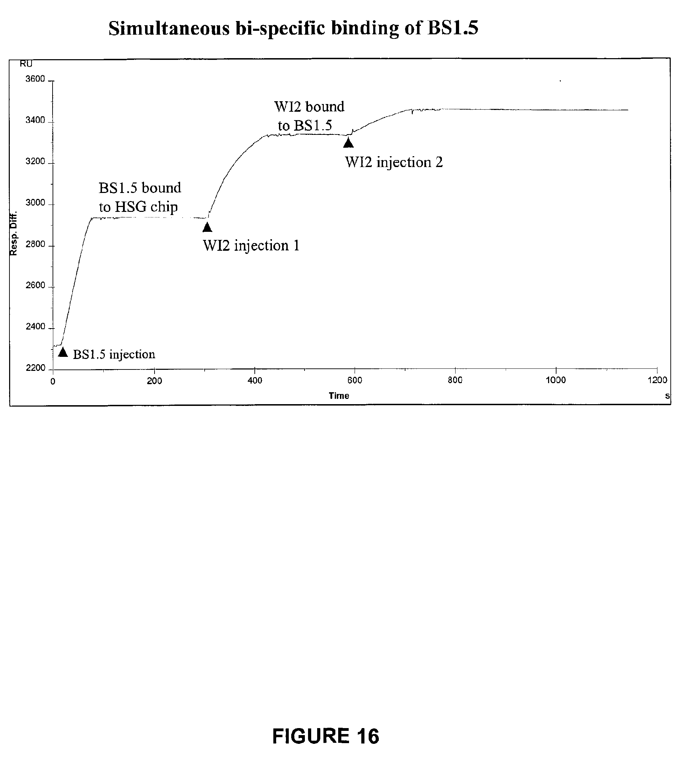

*Figure 16* is a BIAcore sensorgram showing bispecific binding properties of BS1.5 for HSG and WI2. BS1.5 (60ng) was loaded on a high-density HSG-coupled sensor chip and two 400ng injections of the hMN14-binding anti-idiotype MAb, WI2, were allowed to bind to the immobilized BS1.5. Arrows indicate injection times.

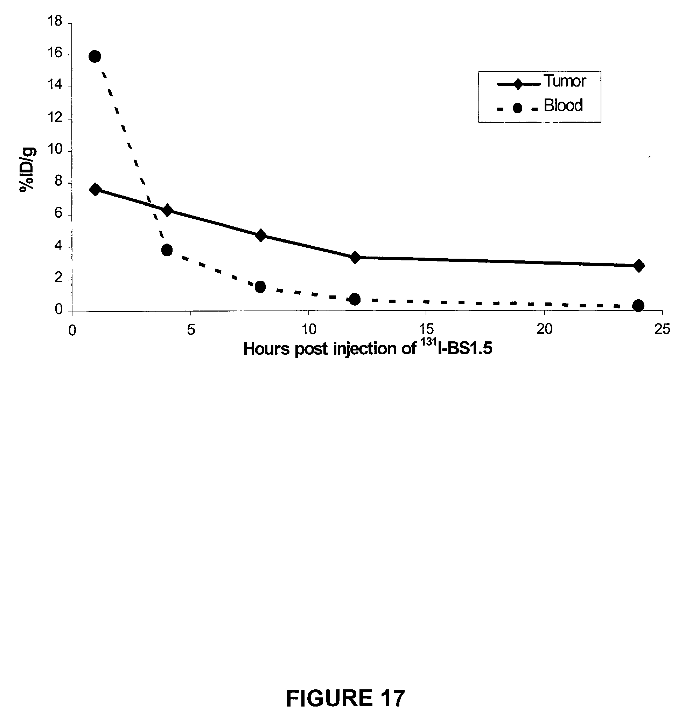

*Figure* 17 shows the levels of ¹³¹I-BS1.5 diabody in the tumor and the blood over the first 96 hours after injection of the diabody. The concentration of ¹³¹I-BS1.5 diabody, measured as the percentage of the injected dose per gram of tissue (%ID/g), is plotted vs. time. Diamonds mark the data points for tumor samples and filled circles mark those of blood samples.

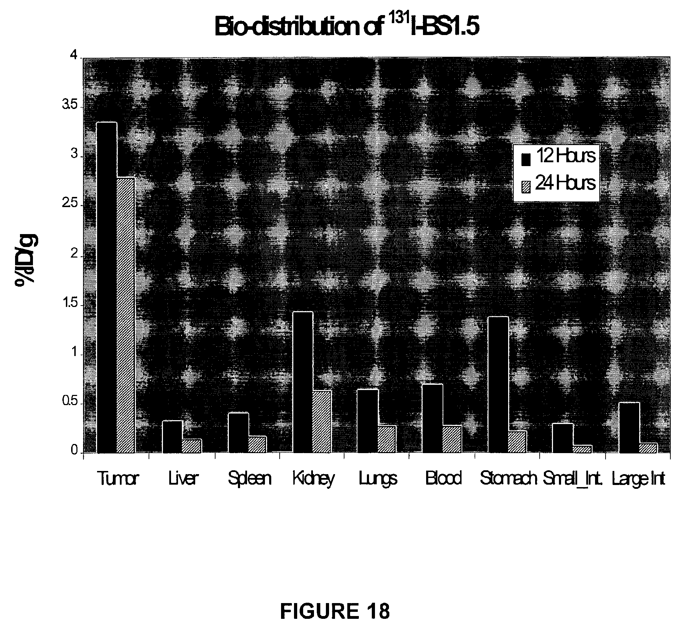

*Figure* 18 shows the biodistribution of ¹³¹I-BS1.5 diabody after 12 and 24 hours post injection in tumor and normal tissue, including liver, spleen, kidney, lungs, blood, stomach, small intestine, and large intestine. The concentration of ¹³¹I-BS1.5 was measured as the percentage of the injected dose per gram of tissue (%ID/g).

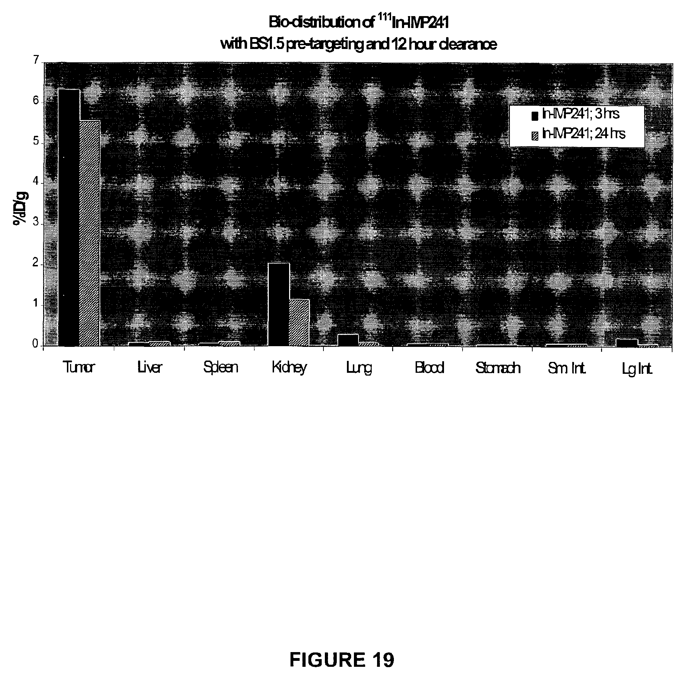

*Figure 19* shows the biodistribution of ¹¹¹In-IMP241 peptide in tumor bearing mice pretargeted with BS1.5. GW39 tumor-bearing nude mice were injected with BS1.5 diabody. After 12 hours of clearance, the ¹¹¹Indium-labeled IMP241 peptide was injected. Radioactivity in the tumor and in normal tissues, including liver, spleen, kidney, lungs, blood, stomach, small intestine, and large intestine, was measured at 3 and 24 hours post injection of ¹¹¹In-IMP241. The concentration of ¹¹¹In-IMP241 was measured as the percentage of the injected dose per gram of tissue (%ID/g).

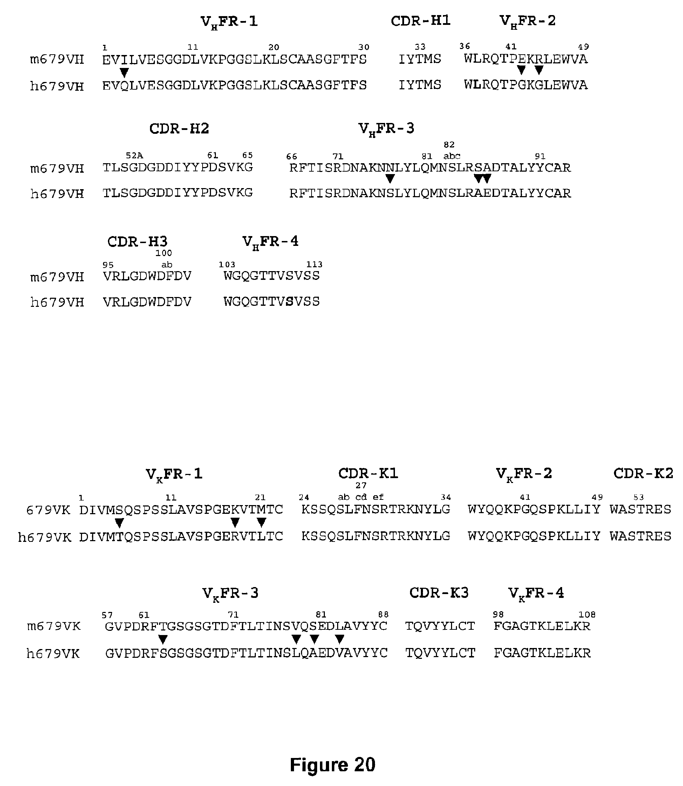

*Figure 20* shows an alignment of murine (m) and humanized (h) 679 V_{H} and V_{K} amino acid sequences using the Kabat numbering scheme. Amino acid substitutions made during humanization are indicated with arrowheads. The CDR and framework regions are indicated.

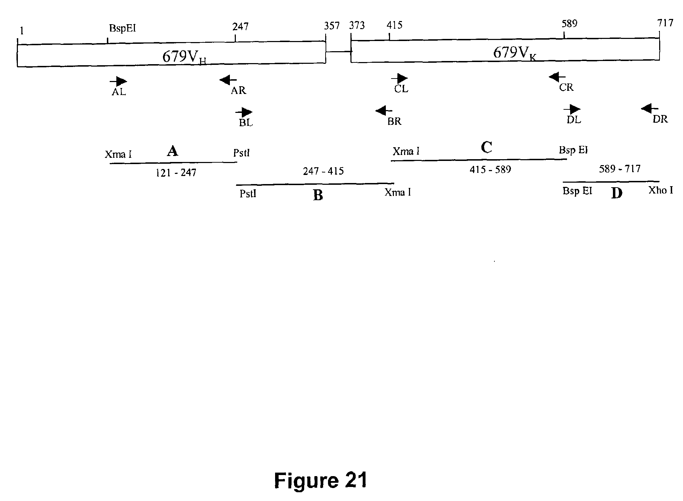

*Figure 21* shows the relative locations of the PCR primers used for humanization of 679scFv-L5. Arrows signify the primers. The intermediate PCR products are also shown (A,B,C and D). All numbering represent nucleic acid positions in 679scFv-L5.

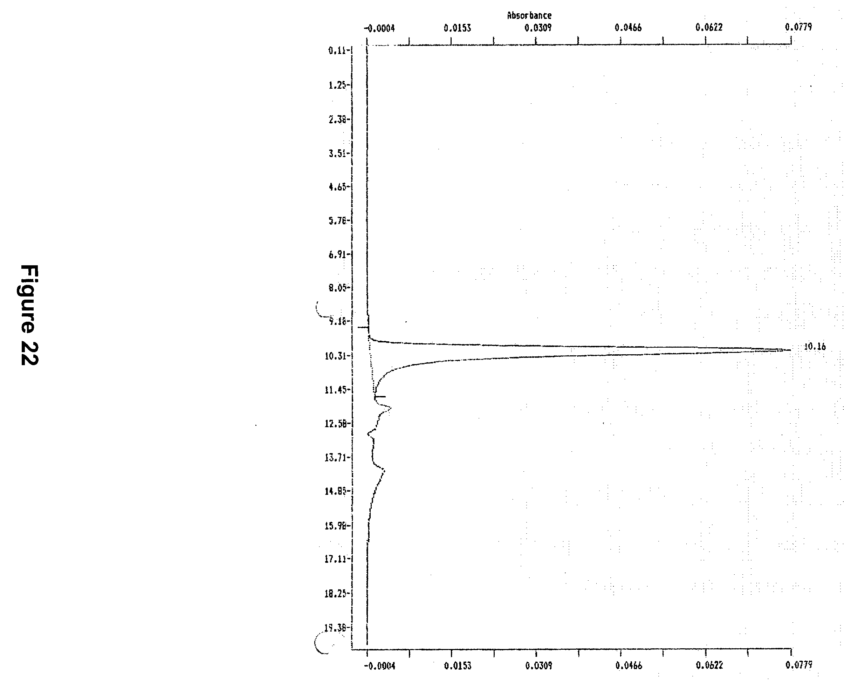

*Figure 22* shows size-exclusion HPLC analysis of the BS1.5H after purification. The HPLC elution peak of BS1.5H is at 10.16 min. Soluble proteins from an induced 5L culture were purified by Ni-NTA IMAC followed by Q-Sepharose anion exchange chromatography. The flow through fraction of the Q-Sepharose column was injected for HPLC analysis.

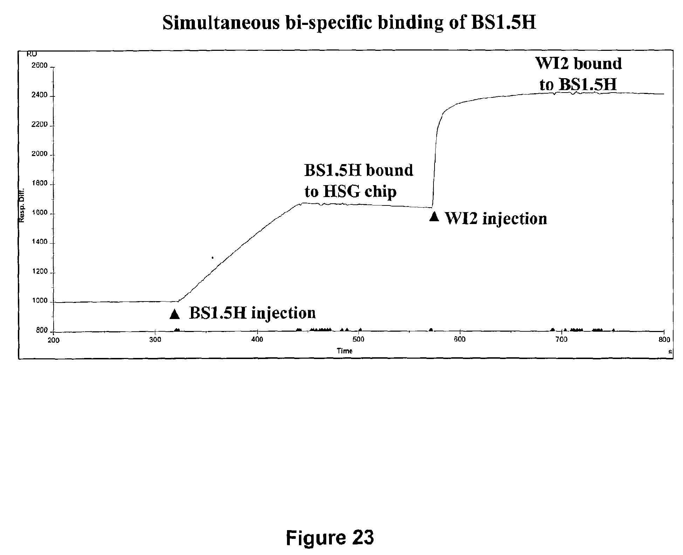

*Figure 23* is a BIAcore sensorgram showing bispecific binding properties of BS1.5H for HSG and WI2. BS1.5H (60ng) was loaded on a high-density HSG-coupled sensor chip and a 1µg injection of the hMN14-binding anti-idiotype MAb, WI2, was allowed to bind to the immobilized BS1.5H. Arrows indicate injection times.

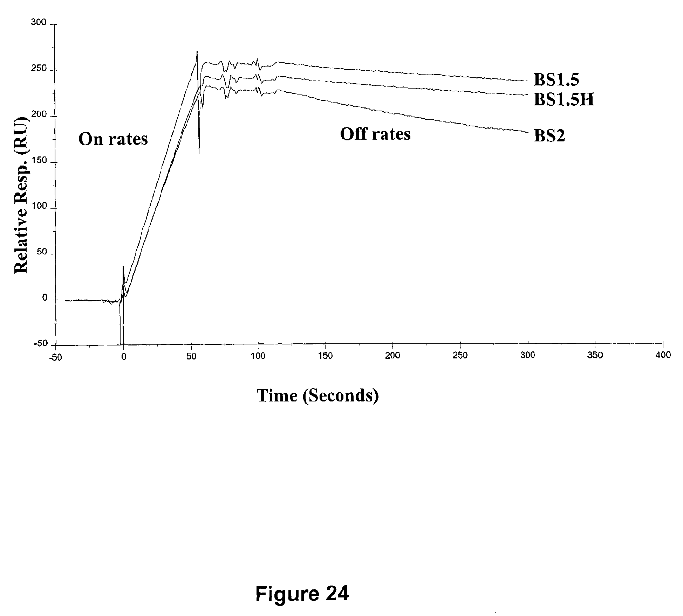

*Figure* 24 shows the comparison of BIAcore binding curves between BS1.5H, BS1.5 and BS2. Similar amounts of the bispecific diabodies were injected on a low density HSG-coupled sensor chip and the resulting binding curves were superimposed.

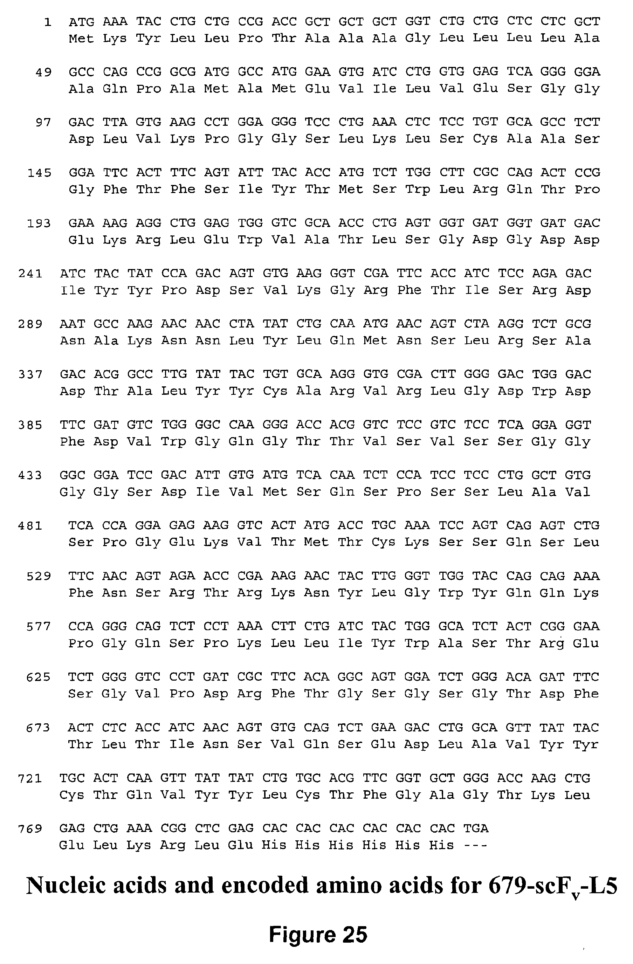

*Figure 25* is the coding sequence of nucleic acids and encoded amino acids for 679-scFᵥ-L5. 1-66 is the coding sequence for the pelB leader peptide. 70-426 is the coding sequence for 679V_{H}. 427-441 is the coding sequence for the linker peptide (GGGGS) 442-780 is the coding sequence for 679V_{K}. 787-804 is the coding sequence for the 6 histidine affinity tag.

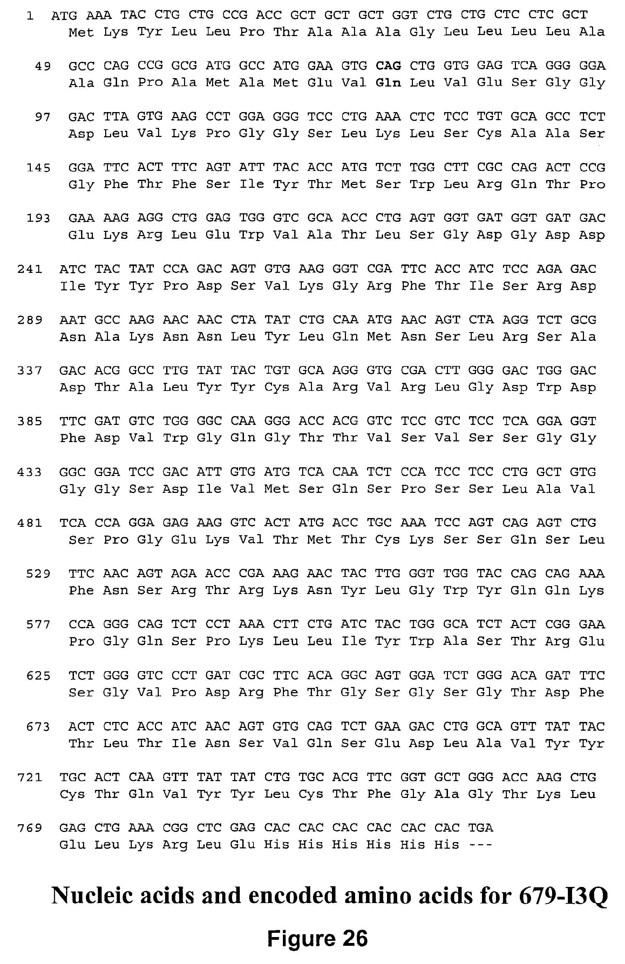

*Figure* 26 is the coding sequence of nucleic acids and encoded amino acids for 679-I3Q. 1-66 is the coding sequence for the pelB leader peptide. 70-426 is the coding sequence for 679 V_{H} (I3Q). 427-441 is the coding sequence for the linker peptide (GGGGS). 442-780 is the coding sequence for 679 V_{K}. 787-804 is the coding sequence for the 6 histidine affinity tag.

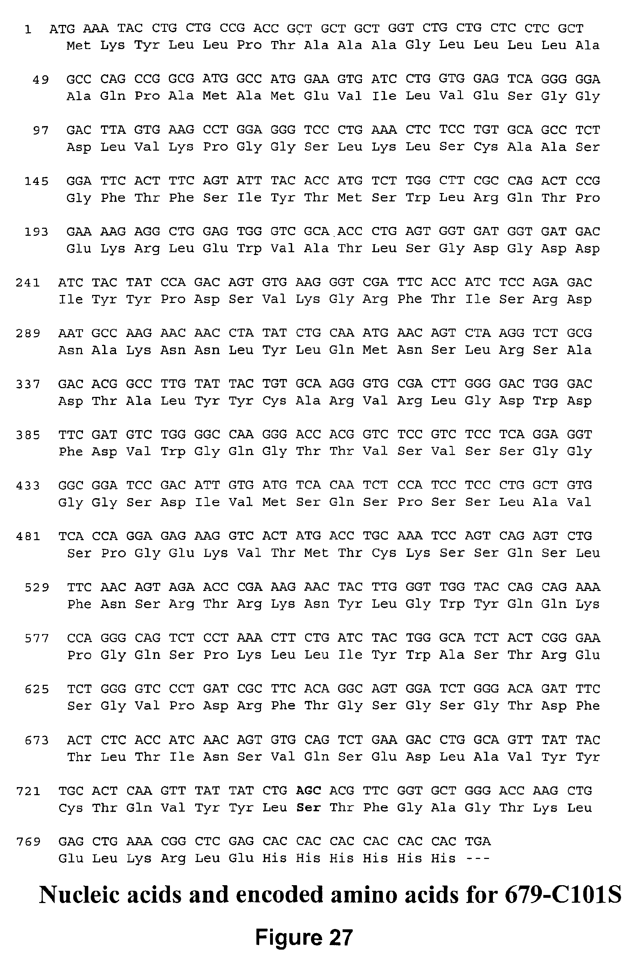

*Figure 27* is the coding sequence of nucleic acids and encoded amino acids for 679-C101S. 1-66 is the coding sequence for the pelB leader peptide. 70-426 is the coding sequence for 679 V_{H}. 427-441 is the coding sequence for the linker peptide (GGGGS). 442-780 is the coding sequence for 679 V_{K} (C101S). 787-804 is the coding sequence for the 6 histidine affinity tag.

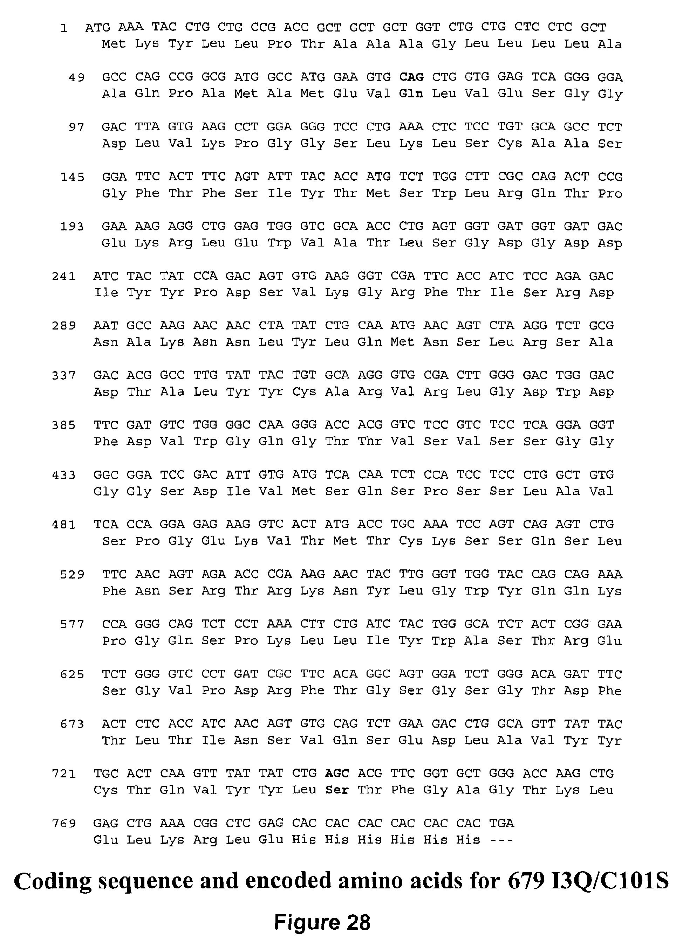

*Figure* 28 is the coding sequence and encoded amino acids for 679 I3Q/C101S.
*Figure* 29 is the coding sequence of nucleic acids and encoded amino acids for hMN14-scFᵥ-L5. 1-66 is the coding sequence for the pelB leader peptide. 70-423 is the coding sequence for hMN14 V_{H}. 424-438 is the coding sequence for the linker peptide (GGGGS). 439-759 is the coding sequence for hMN14 V_{K}. 766-783 is the coding sequence for the 6 histidine affinity tag.
*Figure 30* is the coding sequence of nucleic acids and encoded amino acids for polypeptide #1 of BS1 (679 x hMN14 bispecific diabody: variant 1). 1-66 is the coding sequence for the pelB leader peptide. 70-426 is the coding sequence for 679 V_{H}. 427-441 is the coding sequence for the linker peptide (GGGGS). 442-762 is the coding sequence for hMN14 V_{K}. 769-786 is the coding sequence for the 6 histidine affinity tag.
*Figure 31* is the coding sequence of nucleic acids and encoded amino acids for polypeptide #2 of BS1 (679 x hMN14 bispecific diabody: variant 1). 1-66 is the coding sequence for the pelB leader peptide. 70-423 is the coding sequence for hMN14 V_{H}. 424-438 is the coding sequence for the linker peptide (GGGGS). 439-777 is the coding sequence for 679 V_{K}. 784-801 is the coding sequence for the 6 histidine affinity tag.
*Figure 32* is the coding sequence of nucleic acids and encoded amino acids for polypeptide #1 of BS1.5 (679 x hMN14 bispecific diabody: variant 2). 1-66 is the coding sequence for the pelB leader peptide. 70-426 is the coding sequence for 679 V_{H} (I3Q). 427-441 is the coding sequence for the linker peptide (GGGGS). 442-762 is the coding sequence for hMN14 V_{K}. 769-786 is the coding sequence for the 6 histidine affinity tag.
*Figure 33* is the coding sequence of nucleic acids and encoded amino acids for polypeptide #2 of BS1.5 (679 x hMN14 bispecific diabody: variant 2). 1-66 is the coding sequence for the pelB leader peptide. 70-423 is the coding sequence for hMN14V_{H}. 424-438 is the coding sequence for the linker peptide (GGGGS). 439-777 is the coding sequence for 679V_{K}. 784-801 is the coding sequence for the 6 histidine affinity tag.
*Figure* 34 is the coding sequence of nucleic acids and encoded amino acids for polypeptide #1 of BS2 (679 x hMN14 bispecific diabody: variant 3). 1-66 is the coding sequence for the pelB leader peptide. 70-426 is the coding sequence for 679V_{H} (I3Q). 427-441 is the coding sequence for the linker peptide (GGGGS). 442-762 is the coding sequence for hMN14V_{K}. 769-786 is the coding sequence for the 6 histidine affinity tag.
*Figure 35* is the coding sequence of nucleic acids and encoded amino acids for polypeptide #2 of BS2 (679 x hMN14 bispecific diabody: variant 3). 1-66 is the coding sequence for the pelB leader peptide. 70-423 is the coding sequence for hMN14V_{H}. 424-438 is the coding sequence for the linker peptide (GGGGS). 439-777 is the coding sequence for 679V_{K} C101S. 784-801 is the coding sequence for the 6 histidine affinity tag.

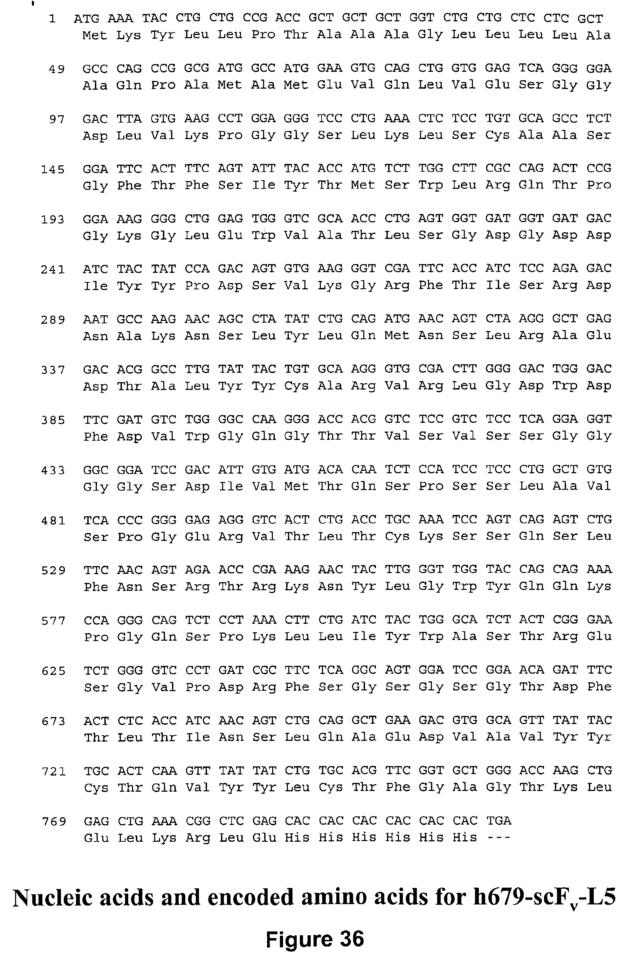

*Figure 36* is the coding sequence of nucleic acids and encoded amino acids for h679-scFᵥ-L5. 1-66 is the coding sequence for the pelB leader peptide. 70-426 is the coding sequence for h679V_{H}. 427-441 is the coding sequence for the linker peptide (GGGGS). 442-780 is the coding sequence for h679V_{K}. 787-804 is the coding sequence for the 6 histidine affinity tag.
*Figure 37* is the coding sequence of nucleic acids and encoded amino acids for polypeptide #1 of BS1.5H (h679 x hMN14 bispecific diabody). 1-66 is the coding sequence for the pelB leader peptide. 70-426 is the coding sequence for h679V_{H}. 427-441 is the coding sequence for the linker peptide (GGGGS). 442-762 is the coding sequence for hMN14V_{K}. 769-786 is the coding sequence for the 6 histidine affinity tag.
*Figure 38* is the coding sequence of nucleic acids and encoded amino acids for polypeptide #2 of BS1.5H (h679 x hMN14 bispecific diabody). 1-66 is the coding sequence for the pelB leader peptide. 70-423 is the coding sequence for hMN14V_{H}. 424-438 is the coding sequence for the linker peptide (GGGGS). 439-777 is the coding sequence for h679V_{K} C101S. 784-801 is the coding sequence for the 6 histidine affinity tag.

### DETAILED DESCRIPTION OF THE PREFERRED EMBODIMENTS

This invention also relates to a multivalent, multi-specific binding protein comprising at least one binding site for a hapten moiety and at least one binding site for a target antigen as defined in the claims. The hapten is connected to a small molecule that carries a diagnostic agent and/or a therapeutic agent. The present disclosure further relates to bispecific diabodies that bind with hapten moieties and target antigens and to recombinant vectors useful for the expression of these functional diabodies in a microbial host.

Structurally, whole antibodies are composed of one or more copies of an Y-shaped unit that contains four polypeptides chains. Two chains are identical copies of a polypeptide, referred to as the heavy chain, and two chains are identical copies of a polypeptide, referred to as the light chain. The two heavy chains are linked together by one or more disulfide bonds and each light chain is linked to one of the heavy chains by one disulfide bond. Each chain has a N-terminal variable domains, referred to as V_{H} and V_{L} for the heavy and the light chains, respectively, and the non-covalent association of a pair of V_{H} and V_{L}, referred to as the Fv fragment, forms one antigen-binding site.

Discrete Fv fragments are prone to dissociation at low protein concentrations and under physiological conditions [Glockshuber et al., Biochemistry (1990) 29: 1362-1367], and therefore are not of much practical use. To improve stability and enhance potential utility, recombinant single-chain Fv (scFv) fragments have been produced and studied extensively, in which the C-terminal of the V_{H} domain (or V_{L}) is joined to the N-terminal of the V_{L} domain (or V_{H}) via a peptide linker of variable length. [For a recent review, see Hudson and Kortt, J. Immunological methods (1999) 231: 177-189].

ScFvs with linkers greater than 12 amino acid residues in length (for example, 15-or 18-residue linkers) allow interaction between the V_{H} and V_{L} domains on the same chain and generally form a mixture of monomers, dimers (termed diabodies) and small amounts of higher mass multimers, [Kortt et al., Eur. J. Biochem. (1994) 221: 151-157]. ScFvs with linkers of 5 or less amino acid residues, however, prohibit intramolecular pairing of the V_{H} and V_{L} domains on the same chain, forcing pairing with V_{H} and V_{L} domains on a different chain. Linkers between 3- and 12-residues form predominantly dimers [Atwell et al., Protein Engineering (1999) 12: 597-604]. With linkers between 0 and 2 residues, trimeric (termed triabodies), tetrameric (termed tetrabodies) or higher oligomeric structures of scFvs are in favor; however, the exact patterns of oligomerization appear to depend on the composition as well as the orientation of the V-domains, in addition to the linker length. For example, scFvs of the anti-neuraminidase antibody NC10 formed predominantly trimers (V_{H} to V_{L} orientation) or tetramers (V_{L} to V_{H} orientation) with 0-residue linkers [Dolezal et al., Protein Engineering (2000) 13: 565-574]. For scFvs constructed from NC10 with 1-and 2-residue linkers, the V_{H} to V_{L} orientation formed predominantly diabodies [Atwell et al., Protein Engineering (1999) 12: 597-604]; in contrast, the V_{L} to V_{H} orientation formed a mixture of tetramers, trimers, dimers, and higher mass multimers [Dolezal et al., Protein Engineering (2000) 13: 565-574]. For scFvs constructed from the anti-CD19 antibody HD37 in the V_{H} to V_{L} orientation, the 0-residue linker formed exclusively trimers and the 1-residue linker formed exclusively tetramers [Le Gall et al., FEBS Letters (1999) 453: 164-168].

As the non-covalent association of two or more identical scFv molecules can form functional diabodies, triabodies and tetrabodies, which are multivalent but monospecific, a similar association of two or more different scFv molecules, if constructed properly, may form functional multispecific scFv multimers. Bispecific diabodies are heterodimers of two different scFvs, each scFv consisting of the V_{H} domain from one antibody connected by a short linker to the V_{L} domain of another antibody. Several bispecific diabodies have been made using a di-cistronic expression vector that contains in one cistron a recombinant gene construct comprising V_{H1}-linker-V_{L2} and in the other cistron a second recombinant gene construct comprising V_{H2}-linker-V_{L1}. [See Holliger et al., Proc. Natl. Acad. Sci. USA (1993) 90: 6444-6448; Atwell et al., Molecular Immunology (1996) 33: 1301-1302; Holliger et al., Nature Biotechnology (1997) 15: 632-631; Helfrich et al., Int. J. Cancer (1998) 76: 232-239; Kipriyanov et al., Int. J. Cancer (1998) 77: 763-772; Holiger et al., Cancer Research (1999) 59: 2909-2916]. More recently, a tetravalent tandem diabody (termed tandab) with dual specificity has also been reported [Cochlovius et al., Cancer Research (2000) 60: 4336-4341]. The bispecific tandab is a homodimer of two polypeptides, each containing four variable domains of two different antibodies (V_{H1}, V_{L1}, V_{H2}, V_{L2}) linked in an orientation to facilitate the formation of two potential binding sites for each of the two different specificities upon self-association. Methods of constructing scFvs are disclosed in U.S. 4,946,778 (1990) and U.S. 5,132,405 (1992). Methods of producing scFv-based agents of multivalency and multispecificity as described above are disclosed in U.S. 5,837,242 (1998), U.S. 5,844,094 (1998) and WO 98/44001 (1998) for bispecific diabodies, and in PCT/DE99/01350 for tandem diabodies.

Alternative methods of manufacturing multispecific and multivalent antigen-binding proteins from V_{H} and V_{L} domains are disclosed in U.S. 5,989,830 and U.S. 6,239,259. Such multivalent and multispecific antigen-binding proteins are obtained by expressing a discistronic vector which encodes two polypeptide chains, with one polypeptide chain consisting of two or more V_{H} domains (from the same or different antibodies) connected in series by a peptide linker and the other polypeptide chain consisting of complementary V_{L} domains connected in series by a peptide linker.

The present disclosure utilizes two monoclonal antibodies, 679 and hMN14, and two point mutations of 679, (679-V_{H} (I3Q) and 679-V_{K}(C101S)), to produce antigen specific diabodies. In addition, a bispecific diabody is produced from hMN14 and h679, which is obtained by grafting the CDRs of 679 onto a framework of amino acid residues found in human antibodies. The murine monoclonal antibody designated 679 (an IgG1, K) binds with high affinity to molecules containing the moiety histamine-succinyl-glycyl (HSG) (Morel et al., Molecular Immunology, 27, 995-1000, 1990). The nucleotide sequence pertaining to the variable domains (V_{H} and V_{K}) of 679 has been determined (Qu *et al.,* unpublished results). V_{K} is one of two isotypes of the antibody light chains, V_{L}. As depicted in Figure 1, the design of the gene construct (679-scFv-L5) for expressing a 679 diabody possesses the following features: 1) The carboxyl terminal end of V_{H} is linked to the amino terminal end of V_{K} by the peptide linker Gly-Gly-Gly-Gly-Ser (G₄S). The use of the G₄S peptide linker enables the secreted polypeptide to dimerize into a diabody, forming two binding sites for HSG. 2) A pelB leader signal peptide sequence precedes the V_{H} gene to facilitate the transport of the polypeptide to the periplasmic space of *E. coli.* 3) Six histidine (His) residues are added to the carboxyl terminus to allow purification by IMAC. The DNA coding sequence and the corresponding encoded amino acids for 679-scFv-L5 are contained in Figure 25 (Seq IDs). The DNA coding sequence and the corresponding encoded amino acids for 679-I3Q are contained in Figure 26 (Seq IDs). The DNA coding sequence and the corresponding encoded amino acids for 679-C101S are contained in Figure 27 (Seq IDs). Figure 1 also includes a stick figure drawing of the mature polypeptide after proteolytic removal of the pelB leader peptide and a stick figure drawing of a 679 diabody, including the HSG binding sites.

Two site-directed point mutations were made to increase the amount of 679 diabodies in soluble extracts. Specifically, converting residue 3 in the 679V_{H} sequence from Ile to Gln (I3Q), or residue 101 in the 679V_{K} sequence from Cys to Ser (C101S), or both (I3Q/C101S), resulted in at least a ten-fold increase in soluble expression levels. Moreover, 679 can be humanized or fully human to help avoid an adverse response to the murine antibody.

hMN14 is a humanized monoclonal antibody (Mab) that binds specifically to CEA (Shevitz et al, J. Nucl. Med., Supp., 34, 217P, 1993; U.S. 6,254,868 (2001)). While the original Mabs were murine, humanized antibody reagents are now utilized to reduce the human anti-mouse antibody response. The variable regions of this antibody were engineered into an expression construct (hMN14-scFv-L5) in a similar fashion to 679-scFv-L5 as described in Example 1. As depicted in Figure 3, the design of the gene construct (hMN14-scFv-L5) for expressing an hMN14 diabody possesses the following features: 1) The carboxyl terminal end of V_{H} is linked to the amino terminal end of V_{K} by the peptide linker Gly-Gly-Gly-Gly-Ser (G₄S). The use of the G₄S peptide linker enables the secreted polypeptide to dimerize into a diabody, forming two binding sites for CEA. 2) A pelB leader sequence precedes the V_{H} gene to facilitate the transport of the polypeptide to the periplasmic space *of E. coli.* 3) Six histidine (His) residues are added to the carboxyl terminus to allow purification by IMAC. The DNA coding sequence and the corresponding encoded amino acids for hMN14-scFv-L5 are contained in Figure 29 (Seq IDs). Figure 3 also shows a stick figure drawing of the mature polypeptide following proteolytic removal of the pelB leader peptide, and a stick figure drawing of a hMN14 diabody, including CEA binding sites.

Di-cistronic expression vectors were constructed through a series of sub-cloning procedures outlined in Figures 8 and 9 and described in Example 6. The di-cistronic expression cassette for bispecific hMN14x679 diabody is shown schematically in Figure 10. The expression cassette may be contained in a plasmid, which is a small, doublestranded DNA forming an extra-chromosomal self-replicating genetic element in many bacteria and some eukaryotes and is widely used in genetic engineering as a cloning vector. A cloning vector is a DNA molecule that can replicate on its own in a microbial host cell. This invention describes a vector that expresses bispecific diabodies. A host cell accepts a vector for reproduction and the vector replicates each time the host cell divides. A commonly used host cell is *Escherichia Coli* (E. Coli), however, other host cells are available.

When the di-cistronic cassette as shown in Figure 10 is expressed in *E. coli,* some of the polypeptides fold and spontaneously form soluble bispecific diabodies. The bispecific diabody shown in Figure 10 forms one binding site having high affinity for HSG and one binding site having high affinity for CEA.

In this instance, the carboxyl terminal end of the V_{H} segment of the 679 MAb is connected to the amino terminal end of the V_{K} segment of the hMN14 MAb by a five amino acid residue linker, and the carboxyl terminal end of the V_{H} segment of the hMN14 MAb is connected to the amino terminal end of the V_{K} segment of the 679 MAb by the same five amino acid residue linker. Three variants of 679 x hMN14 bispecific diabodies have been produced and tested. BS1 is composed of the wild-type sequences for both 679 and hMN14 variable regions. BS1.5 incorporates the 679V_{H} I3Q mutation. BS2 incorporates both the 679V_{H} I3Q and the 679V_{K} C101S mutations. The DNA coding sequences and the corresponding encoded amino acids for the two polypeptides of BS1, BS1.5, and BS2 are contained in Figures 30 & 31, 32 & 33, and 34 & 35 (Seq IDs), respectively. Additionally, a bispecific diabody of h679xhMN14 has been constructed and named BS1.5H (See Figures 37 & 38).

The ultimate use of these bispecific diabodies is for pre-targeting CEA positive tumors for subsequent specific delivery of therapeutic radioisotopes carried by HSG containing peptides. These diabodies bind selectively to targeted antigens and when combined with a bivalent di-HSG hapten allow for increased affinity and a longer residence time at the desired location. Moreover, non-antigen bound diabodies are cleared from the body quickly and exposure of normal tissues is minimized.

Delivering a diagnostic or a therapeutic agent to a target for diagnosis or treatment by means as defined in the claims includes administering a patient with the binding protein, waiting a sufficient amount of time for an amount of the non-binding protein to clear the patient's blood stream, and administering a diagnostic or therapeutic agent that binds to a binding site of the binding protein. Diagnosis further requires the step of detecting the bound proteins with known techniques. The diagnostic or therapeutic carrier molecule comprises a diagnostically or therapeutically efficient agent, a linking moiety, and one or more hapten moieties. The hapten moieties are positioned to permit simultaneous binding of the hapten moieties with the binding protein.

Administration of the binding protein and diagnostic or therapeutic agents of the present invention to a mammal may be intravenous, intraarterial, intraperitoneal, intramuscular, subcutaneous, intrapleural, intrathecal, by perfusion through a regional catheter, or by direct intralesional injection. When administering the binding moiety by injection, the administration may be by continuous infusion or by single or multiple boluses.

The unmixed diagnostic or therapeutic agent and bispecific antibody may be provided as a kit for human therapeutic and diagnostic use in a pharmaceutically acceptable injection vehicle, preferably phosphate-buffered saline (PBS) at physiological pH and concentration. The preparation preferably will be sterile, especially if it is intended for use in humans. Optional components of such kits would normally be containers of stabilizers, buffers, labeling reagents, radioisotopes, paramagnetic compounds, second antibody for enhanced clearance, and conventional syringes, columns, vials and the like.

### EXAMPLES

The examples below are illustrative of embodiments of the current invention and should not be used, in any way, to limit the scope of the claims.

### Example 1 - Construction of Plasmids for expression of 679 diabody in E. coli

Standard recombinant DNA methods were used to obtain 679-scFv-L5 as follows. A plasmid containing the V_{H} sequence of 679 was used as the template for polymerase chain reaction (PCR) using Pfu polymerase and the two oligonucleotide primers specified below:
**679V_{H}-Left** 5'-TCAGCCATGGAAGTGATCCTGGTGGAGTCAGGGGGAGACT-3'
**679V_{H}-Right (G₄S)** 5'-TGAGGATCCGCCACCTCCTGAGGAGACGGAGACCGTGGTC-3'

The left PCR primer contains a 5' NcoI restriction site. The right PCR primer contains the sequence for a 5 amino acid residue linker (G₄S) and a BamHI restriction site. The PCR product was digested with NcoI and BamHI and ligated in frame with the pelB leader sequence into NcoI/BamHI digested pET-26b vector (Novagen) to generate 679V_{H}L5-pET26.

A plasmid containing the V_{K} sequence of 679 was used as the template for PCR using Pfu polymerase and the two oligonucleotide primers specified below:
**679V_{K}-Left** 5'-CTGAGGATCCGACATTGTGATGTCACAATCT-3'
**679V_{K}-Right** 5'- ATCCTCGAGCCGTTTCAGCTCCAGCTTGGT-3'

The left and right PCR primers contain BamHI and XhoI restriction sites, respectively. The PCR product was digested with XhoI and BamHI and ligated (in frame with the 679V_{H}, G₄S linker, and 6His sequences) into the XhoI/BamHI digested 679V_{H}L5-pET26 to generate the expression construct 679-scFv-L5. The DNA sequence of the inserted gene confirmed that the V_{H} and V_{K} sequences were identical to those of the original cDNA clones and the sequences of the ligation sites and linker regions were as designed. The gene construct, 679-scFv-L5, is illustrated in Figure 1.

### Example 2 - Expression of 679 diabody in E. coli

Competent *E. coli* BL21(P-Lys-S) cells were transformed with 679-scFv-L5 by standard methods. Cultures were shaken in 2xYT media supplemented with 100µg/ml kanamycin sulphate and 34µg/ml chloramphenicol and grown at 37°C to OD₆₀₀ of 1.6 - 1.8. An equal volume of room temperature 2xYT media supplemented with antibiotics and 0.8M sucrose was added to the cultures, which were then transferred to 20°C. After 30 minutes at 20°C, expression was induced by the addition of 40µM IPTG and continued at 20°C for 15-18 hours.

The expression of 679 diabody was examined in (1) cell culture conditioned media, (2) soluble proteins extracted under non-denaturing conditions from the cell pellet following centrifugation, and (3) insoluble material remained in the pellet following several cycles of extraction and centrifugation.

Soluble proteins were extracted from bacterial cell pellets as follows. Pellets were frozen and thawed then re-suspended in lysis buffer (2% Triton-X 100; 300mM NaCl; 10mM imidazole; 5mM MgSO₄; 25 units/ml benzonase; 50mM NaH₂PO₄, pH 8.0) using an amount equal to 1 % of the culture volume. The suspension was homogenized by sonication , clarified by centrifugation, and loaded onto Ni-NTA IMAC columns. After being washed with buffer containing 20mM imidazole, the columns were eluted with 100mM imidazole buffer (100mM imidazole; 50mM NaCl; 25mM Tris, pH 7.5) and the eluate obtained was further purified on a Q-Sepharose column.

The insoluble material was solubilized in denaturing Ni-NTA binding buffer (8M urea; 10mM imidazole; 0.1M NaH₂PO₄; 10mM Tris, pH 8.0) and mixed with 1ml of Ni-NTA agarose (Qiagen, inc.). The mixture was rocked at room temperature for 1 hour then the resin was washed once with 50ml of the same buffer and loaded onto a column. The column was washed with 20ml of the same buffer followed by 20ml of wash buffer (8M urea; 20mM imidazole; 0.1M NaH₂PO₄; 10mM Tris, pH 8.0). Bound proteins were eluted with 5ml of denaturing elution buffer (8M urea; 250mM imidazole; 0.1M NaH₂PO₄; 10mM Tris, pH 8.0).

As shown by the results of reducing SDS-PAGE in Figure 2, a robust induction was evident in the whole cell lysate (lane 2), which displayed a predominant band corresponding to a protein of approximately 28 kD (the predicted MW for 679 scFv). However, virtually all of the induced protein was present in the insoluble fraction (lane 5), and none was detected in the 10x concentrated culture media (lane 3). The induced protein was purified from the insoluble fraction following solubilization and elution of the bound material off a Ni-NTA column under denaturing conditions (lane 1). The soluble extract contained a trace amount of HSG-binding material, estimated to be about 1 ug per liter of culture by BIAcore analysis.

### Example 3 - 679 Diabodies formed from scFv mutants

Two site-directed point mutations were made to increase the amount of 679 diabodies in soluble extracts. Specifically, converting residue 3 in the 679V_{H} sequence from Ile to Gln (I3Q), or residue 101 in the 679V_{K} sequence from Cys to Ser (C101S), or both (I3Q/C101S), resulted in at least a ten-fold increase in soluble expression levels. The mutations were introduced in synthetic oligonucleotides used for PCR. The V_{H}-I3Q mutation was incorporated in the oligonucleotide primer depicted below:
**679V_{H} I3Q-Left** 5'- CCATGGAAGTGCAGCTGGTGGAGTCAGGG-3'

This primer was paired with 679V_{H}-Right (Example 1) to generate the V_{H}-I3Q mutant by PCR from 679-scFv-L5 template using Pfu polymerase.

The 679V_{K}-C101S mutation was incorporated in the oligonucleotide primer specified below:
**679V_{K} C101S-Right**

5’GCTCGAGCCGTTTCAGCTCCAGCTTGGTCCCAGCACCGAACGTGCT
CAGATAATAAACTTGAG-3’

This primer was paired with 679-V_{K} Left (Example 1) to generate 679V_{K}-C101S mutant by PCR from 679-scFv-L5 template using Pfu polymerase. The PCR products were cloned into pET26b following the same procedure as described above in Example 1.

Expression levels in the soluble fractions were estimated by BIAcore analysis using a HSG coupled sensor chip. The expression levels of I3Q, C101S, or I3Q/C101S mutant 679 diabody were about 10 ug/L as compared to about 1 ug/L for the wild type.

### Example 4 - Construction of plasmids for expression of hMN14 diabody in E. coli

Standard recombinant DNA methods were used to obtain hMN14-scFv-L5 as follows. The hMN14 V_{H} and V_{K} sequences were amplified from a vector constructed for expressing hMN14 Fab' (Leung et al., Cancer Research, Supp., 55, 5968s-5972s, 1995) by PCR with Pfu polymerase. The hMN14V_{H} sequence was amplified using the oligonucleotide primers specified below:
**hMN14V_{H}-Left** 5'- CGTACCATGGAGGTCCAACTGGTGGAGA-3'
**hMN14V_{H}-Right (G₄S)** 5'-CATAGGATCCACCGCCTCCGGAGACGGTGACCGGGGT-3'

The left PCR primer contains a 5' NcoI restriction site. The right PCR primer contains a sequence for a 5 amino acid residue linker (G₄S) and a BamHI restriction site. The PCR product was digested with NcoI and BamHI and ligated, in frame with the pelB leader sequence, into NcoI/BamHI digested pET-26b vector to generate hMN14V_{H}L5-pET26. The hMN14V_{K} sequence was amplified using the oligonucleotide primers specified below:
**hMN14V_{K}-Left** 5'- CTGAGGATCCGACATCCAGCTGACCCAGAG-3'
**hMNI4V_{K}-Right** 5'-GCTACTCGAGACGTTTGATTTCCACCTTGG-3'

The left and right PCR primers contain BamHI and XhoI restriction sites, respectively. The PCR product was digested with XhoI and BamHI and ligated, in frame with the hMN14V_{H}, G₄S linker and 6His sequences, into the XhoI/BamHI digested hMNI4V_{H}L5-pET26 construct to generate the expression construct hMN14-scFv-L5. The DNA sequence of this construct was verified by automated DNA sequencing. The gene construct, hMN14-scFv-L5, is illustrated in Figure 3.

### Example 5 - Expression of hMN14 diabody in E. coli

The hMN14-scFv-L5 construct was used to transform BL21(P-LysS) *E. coli.* Culture conditions, induction, and purification were carried out similar to those described for the 679 diabody in Example 1, except that the hMN14 diabody was purified by affinity chromatography, instead of Q-Sepharose anion exchange chromatography, via binding to an anti-id antibody immobilized on Affi-gel. Soluble proteins that bound and eluted from Ni-NTA resin were loaded on a WI2 anti-idiotype affinity column. The column was washed with PBS and the product was eluted with 0.1M Glycine; 0.1M NaCl, pH 2.5 and neutralized immediately.

Although most of the hMN14scFv expressed was present as insoluble protein, approximately 1.5 mg/L culture of soluble hMN14scFv was purified from the soluble fraction. As shown by size-exclusion high performance liquid chromatography (HPLC), a predominant peak was observed in Figures 4A and 4B at 9.8 min for the IMAC purified as well as the affinity purified material. The retention time of hMN14 Fab', which has a molecular weight of approximately 50 kDa, was 9.75 minutes as indicated on the x-axis of Figure 4B. The very similar retention time of hMN14scFv indicates that it exists in solution as a dimer or diabody since the calculated molecular weight of the monomeric hMN14scFv is 26kDa. SDS-PAGE gel analysis in Figure 5A shows a single band of the predicted Mr at 26kDa, and the isoelectric focusing (IEF) gel analysis in Figure 5B yields a band with pI of 8.2, close to the calculated pI of 7.9. A competitive ELISA showed that the hMN14 diabody is functionally active and displays excellent binding properties.

Nude mice bearing the CEA positive GW39 tumor were injected with ¹³¹I-labeled hMN14 diabody and the biodistribution was analyzed at various times post injection. While a significant amount of the diabody remained associated with the tumor for more than 96 hours, much of the free diabody cleared the blood rapidly as illustrated in Figure 6. Figure 7 shows the percentage of the injected dose that is associated with the tumor and with normal tissues, such as liver, spleen, kidney, lungs, blood, stomach, small intestine, and large intestine, at 48 hours after the injection. The amount of the injected dose in each normal tissue is very low when compared to the amount in the tumor. Table 1 summarizes the relative amounts of activity found in normal tissues compared to that in the tumor at 24, 48 and 72 hours.

**Table 1. Tumor to non-tumor ratios**

| | 24hrs | 48hrs | 72hrs |
|---|---|---|---|
| **Tumor** | 1.00 | 1.00 | 1.00 |
| **Liver** | 22.47 | 31.85 | 28.32 |
| **Spleen** | 25.41 | 39.51 | 41.03 |
| **Kidney** | 9.12 | 12.12 | 10.54 |
| **Lung** | 15.49 | 25.70 | 31.75 |
| **Blood** | 9.84 | 17.32 | 21.80 |
| **Stmach** | 9.98 | 17.50 | 23.13 |
| **sm. Int.** | 37.23 | 65.60 | 50.58 |
| **Lg. Int.** | 35.87 | 66.54 | 45.66 |

### Example 6 - 679 x hMN14 bispecific diabody (BS1, BS1.5 and BS2)

### Construction of PET-ER

Before proceeding to expression vectors that direct the synthesis of bispecific diabodies capable of binding to both HSG and CEA, a new vector (pET-ER) was generated by the addition of a multiple cloning site, MCS2, shown in Figure 8A, into the pET-26b vector, shown in Figure 8B. Two complimentary oligonucleotides were synthesized and phosphorylated with T4 polynucleotide kinase. The oligonucleotides were mixed in equal molar concentrations, heated to 95°C then allowed to anneal as the mixture was slowly cooled to room temperature. The duplex structure, MCS2, was ligated into the BlpI restriction site of pET-26b to generate the pET-ER vector as illustrated in Figure 8C. This vector facilitates the construction of di-cistronic expression cassettes and allows for stoichiometric expression of two heterologous polypeptides in a single *E. coli* cell.

### Construction and expression of 679xhMN14 diabodies in E. coli

The di-cistronic expression vectors were constructed through a series of sub-cloning procedures that are outlined in Figure 9. Initially, the V_{K} sequences of 679-scFv-L5 and hMN14-scFv-L5 were exchanged by excision with BamHI and XhoI to generate two intermediate constructs in pET26b. A DNA fragment containing the sequence 679V_{H}-L5-hMN14V_{K}, excised from a pET26b construct with NcoI and XhoI, was ligated into the same restriction sites in pET-ER vector to generate an intermediate clone (679V_{H}-L5-hMN14V_{K}- pET-ER). A 900bp DNA fragment, which includes a ribosomal binding site in addition to the coding sequence for polypeptide 2 (below), was excised from hMN14V_{H}-L5-679V_{K}- pET26b with XbaI and BlpI. This fragment was ligated into the SpeI and BlpI restriction sites of 679V_{H}-L5-hMN14V_{K}- pET-ER to create the final bispecific expression constructs. The di-cistronic expression cassette for bispecific hMN14x679 diabody is shown schematically in Figure 10. The DNA coding sequence of nucleic acids and the corresponding encoded amino acids for the first and second polypeptide sequences of BS1, BS1.5, and BS2 are contained in Figures, 30 & 31, 32 & 33, and 34 & 35 (Seq IDs), respectively. The di-cistronic expression cassette codes for two polypeptides that are arranged as follows:
**Polypeptide 1** Pel B; 679V_{H}; GGGGS linker; hMN14V_{K}; 6 His
**Polypeptide 2** Pel B; hMN14V_{H}; GGGGS linker; 679V_{K}; 6His

When this cassette is expressed in *E. coli,* some of the polypeptides fold and spontaneously form soluble bispecific diabodies. The bispecific diabody, having four polypeptides interacting with each other, is shown in Figure 10. In this instance, the carboxyl terminal end of the V_{H} segment of the 679 MAb is connected to the amino terminal end of the V_{K} segment of the hMN14 MAb by a five amino acid residue linker, and the carboxyl terminal end of the V_{H} segment of the hMN14 MAb is connected to the amino terminal end of the V_{K} segment of the 679 MAb by the same five amino acid residue linker. Each chain forms one half of the 679xhMN14 diabody. The three constructs for expression of 679 x hMN14 bispecific diabodies, BS1, BS1.5, and BS2 were expressed and purified as described for 679scFv in Example 1. The results are described in detail below for BS1.5.

Following IPTG induction, BS1.5-transformed *E. coli* (BL21-pLysS) cultures expressed 0.5 mg of soluble bispecific diabody per liter of culture. From 5L induction, 2.4 mg of highly purified BS1.5 diabody was isolated following the procedures similar to those described in Example 1. Soluble cell extracts were loaded onto a 4ml of Ni-NTA agarose column (Qiagen), which was washed with 20 bed volumes of 10mM imidazole buffer and 5 bed volumes of 20mM imidazole buffer. The diabody was eluted from the IMAC column in 15 ml of 100mM imidazole elution buffer. The eluate was directly passed over a 4-ml Q-Sepharose anion exchange column and the highly purified BS1.5 was collected in the flow through fraction. HPLC analysis showed a single peak illustrated in Figure 11 with a retention time of 9.2 minutes demonstrating that the two heterologous polypeptides, 679V_{H}-GGGGS-hMN14V_{K} and hMN14V_{H}-GGGGS-679V_{K}, exclusively form a dimer or diabody. The purity of the three 679xhMN14 bispecific diabodies was further demonstrated by reducing SDS-PAGE and IEF. A single protein band is seen in Figure 12 at approximately 27 kDa in a Coomassie blue-stained SDS-PAGE gel for BS2. The two polypeptides essentially co-migrate, since their calculated MWs are 26.5 kDa and 27.2 kDa. On IEF gel, as shown in Figure 13, BS1, BS1.5 and BS2 each shows the presence of a single band with a pI of approximately 8.3, which is close to the predicted pI of 7.9 for the three bispecific diabodies.

The binding kinetics of BS1.5 was evaluated by BIAcore using a low density HSG-coupled sensor chip. Binding sensograms were obtained for BS1.5 concentrations from 0 to 54 nM and the resulting data were analyzed with the BIAcore BiaEvaluation software using 1:1 Langmuir binding model, yielding an association constant of the interaction, K_{d}, of 2.4 nM for the binding of BS1.5 to immobilized HSG. Figure 14 shows the BIAcore binding curves at various concentrations of BS1.5. Using the same method, a chemically prepared 679 x hMN14 F(ab')₂ conjugate yields a K_{d} of 1.55 nM. The binding properties for BS1.5 as compared to BS1 and BS2 are summarized in Table 2. A lower K_{d} suggests a higher affinity to the antigen. BS1.5 has the lowest K_{d} and therefore exhibits the greatest affinity to HSG. K_{d} is a measure of the ratio of the off rate and on rate constants, K*_{off}* and K*ₒₙ*, where K_{d}=K*_{off}*/K*ₒₙ.*

**Table 2. Properties of bispecific diabodies**

| | K_{d} | k*ₒₙ* (1/Ms) | k*_{off}* (1/s) | Expression |
|---|---|---|---|---|
| **BS1** | 4.7nM | 2.12e5 | 1.01e-3 | 0.25 mg/L |
| **BS1.5** | 2.5nM | 4.05e5 | 1.01e-3 | 0.5 mg/L |
| **BS2** | 10.6nM | 3.58e5 | 3.81e-3 | 1.0 mg/L |

The binding of BS1.5 to CEA was demonstrated by competitive ELISA. Microtiter plates were coated with 0.5 µg/well with soluble CEA (Scripps Laboratories). BS1.5 at concentrations ranging from 4 - 500 nM were allowed to compete for CEA binding with HRP-conjugated hMN14 IgG (1nM). BS1.5 shows a competitive binding curve similar to that of the 679 x hMN14 F(ab')₂ chemical conjugate. These data indicate that the BS1.5 has a CEA binding affinity similar to the parental hMN14 antibody. The bispecific binding properties of BS1.5 was also analyzed by BIAcore with a high-density HSG-coupled biosensor chip. BS1.5 was pre-bound to the sensor chip before injection of an anti-idiotype MAb designated WI2 that is highly specific for hMN14. Soluble CEA was also used in place of WI2 and gave similar results. As shown in Figure 16, injection of 60 ng of BS1.5 gave a relative response of 620 RU. Subsequent injection of 400ng of WI2 increased the signal by 400 RU. Binding approached saturation with a second WI2 injection (400ng), as a total of 520 RU were added to the 620RU signal of BS1.5. Injection of WI2 following pre-binding with 679 F(ab')₂ or without pre-binding yielded a negligible response. These data demonstrate that BS1.5 has the capability of binding HSG and CEA simultaneously.

BS1 and BS2 each differ from BS1.5 by single point mutations in the 679 component of the diabody. Some of the properties of these molecules are summarized in Table 2. ELISA experiments demonstrate that each of these proteins exhibits similar CEA binding properties, which is not surprising given that the hMN14 component of the diabody is consistent among the three diabodies. Further, BS1 and BS2 are demonstrated by BIAcore analysis to be bispecific and capable of binding to CEA and HSG simultaneously. BS1.5 includes the 679V_{H}I3Q mutation that is not included in BS1, which is composed entirely of the wildtype sequences. This mutation doubles the yield of soluble diabody that is expressed without compromising the binding affinity for HSG. BS2 includes the additional 679V_{K} C101S mutation as well as the 679V_{H} I3Q. With this second change, soluble BS2 is expressed at twice the level of BS1.5, however, the binding affinity for HSG decreased measurably.

### Example 7 - In Vivo Targeting

The potential of these bispecific diabodies for use as pre-targeting CEA positive tumors for subsequent specific delivery of therapeutic radioisotopes carried by HSG containing peptides is demonstrated by BS1.5. Nude mice bearing GW39 (CEA positive) tumors were pre-targeted with BS1.5. Initially, the bio-distribution was followed with ¹³¹I-labeled BS1.5. The results are shown in Figure 17. The diabody rapidly accumulated in the tumor within one hour and slowly cleared. The diabody also accumulated in the blood within one hour, however, significant blood clearance occurred within 8 to 12 hours. At 12 and 24 hour clearance times, the tumors were enriched appreciably with ¹³¹I-BS1.5 as compared to normal tissues, such as liver, spleen, kidney, lungs, blood, stomach, small intestine, and large intestine, as illustrated in Figure 18. Pre-targeting experiments were performed with 12 or 24 hour clearance times following injection of BS1.5 (unlabeled). IMP241, a peptide containing two HSG groups and a DOTA moiety, was loaded with ¹¹¹Indium and injected in BS1.5 pre-targeted mice. The bio-distribution of the ¹¹¹In-IMP241 was examined at 3 and 24 hours after injection. Figure 19 shows the activity in the tumor and normal tissues in pre-targeted mice with 12 hour clearance. Substantial radioactivity accumulates in tumors within 3 hours with only minimal loss after 24 hours. Small amounts of radioactivity was detected in all normal tissues besides the kidney at both time points, suggesting that the diabody is specific to the tumor and radioactive isotopes, but avoids uptake into normal tissues. The tumor to non-tumor ratios of ¹¹¹In-IMP241 are summarized in Table 3.

**Table 3. Tumor to non-tumor ratios for ¹¹¹In-IMP241 after BS1.5 injection and 12- hour clearance.**

| | 3 hrs post ¹¹¹I-IMP241 | 24 hrs post ¹¹¹I-IMP241 |
|---|---|---|
| **Tumor** | 1.00 | 1.00 |
| **Kidney** | 3.30 | 4.96 |
| **Liver** | 82.34 | 60.66 |
| **Spleen** | 179.23 | 67.79 |
| **Lung** | 28.57 | 74.75 |
| **Blood** | 154.78 | 157.18 |
| **Stomach** | 494.84 | 328.03 |
| **Sm. Int.** | 132.72 | 184.14 |
| **Lg Int.** | 34.60 | 172.47 |

### Example 8 - Humanization of 679 V domains

A humanized version of 679-based diabody has been generated that exhibits HSG binding affinity comparable to the murine forms. The strategy employed was to retain all CDR residues and those residues known to interact with the CDR residues while substituting only those residues of the mouse frameworks that are not found in the database of human frameworks at corresponding positions. In such cases if more than one amino acid residue of the human frameworks is known for the same position, the most common one is selected for humanization.

The amino acid sequence for each of the framework regions of m679V_{H} or m679V_{K} were used to query the NCBI database and aligned with human antibody (h-Ab) sequences. Most amino acid residues of the murine 679 frameworks are identical with some or all of the human frameworks in the database at corresponding positions and therefore they are conserved for h679. For those amino acid residues of the murine 679 frameworks that are not found in any of the human frameworks, they are substituted with the most common residue found in the homologous h-Abs at the corresponding positions. However, if a residue in a particular position is likely to interact with the CDRs or to be involved in the V_{H} and V_{K} association (E.A. Padlan, Molecular Immunology, 31, 169-217, 1994), the residue in m679 is retained in h679.

### Substitutions

Figure 20 shows an alignment of m679 and humanized h679. The Kabat numbering system is used and framework regions (FR) as well as CDRs are indicated. Arrows signify amino acid substitutions. For all of the considerations below, human sequences with high levels of sequence identity were compared to m679.

### V_{H} framework region 1 (V_{H}FR-1)

All but one of the m679 V_{H}FR-1 amino acids is commonly found in the h-Abs and were therefore left unchanged in h679. At position V_{H}-3, glutamine (Q), which is always in this position in the h-Abs, was substituted for isoleucine (I), which is not found in the h-Abs. The V_{H}I3Q substitution has previously been introduced into both m679 diabodies and bi-specific diabodies and was found to increase the solubility levels of expressed products.

### V_{H} framework region 2 (V_{H}FR-2)

This region is small yet divergent. Residues found in three positions in V_{H}FR-2 of m679 are not found in h-Abs. In m679, leucine (L) is in position V_{H}-37, which in h-Abs is almost always valine. However, the leucine was retained in h679 because this position is known to be strongly involved in V_{H} to V_{K} association and often is in contact with CDR residues. Positions V_{H} 42 and 44 are always glycine in the h-Abs and do not contact the V_{K} or CDRs. Therefore, glutamic acid (E) at V_{H}-42 and arginine (R) at V_{H}-44 were each substituted with glycine.

### V_{H} framework region 3 (V_{H}FR-3)

Substitutions at three of 32 positions in V_{H}FR-3 made this region of h679 entirely humanized. None of the three positions are known to be involved in V_{H}-V_{K} or CDR contact so the following substitutions were made with the most common h-Ab amino acid for the respective positions; serine (S) for asparagine (N) at V_{H}-77; alanine (A) for serine (S) at V_{H}-84; and glutamic acid (E) for alanine (A) at V_{H}-85.

### V_{H} framework regions 4 (V_{H}FR-4)

Substitution of threonine (T) for serine (S) at V_{H}-110 would make this region completely humanized. However, for technical reasons, we chose to keep T in the V_{H}FR-4 of h679.

### V_{K} framework region 1 (V_{K}FR-1)

This region has considerable variability amongst the h-Abs. The m679 amino acids at 20 of the 23 positions in V_{K}FR-1 are acceptable for h-Abs. The following substitutions were made at three positions with the most common h-Ab amino acid for the respective positions: threonine (T) for serine (S) at V_{K}-5; arginine (R) for lysine (K) at V_{K}-18; and leucine (L) for methionine (M) at V_{K}-21. These positions are not known to be involved in V_{H}-V_{K} or CDR contacts.

### V_{K} framework region 2 (V_{K}FR-2)

This short region resembles the human sequences and is acceptable as is.

### V_{K} framework region 3 (V_{K}ER-3)

This large (31 amino acids) region requires four substitutions for complete humanization. Serine (S), always found in h-Abs at V_{K}-63, replaced threonine (T). Leucine (L), always found in h-Abs at V_{K}-78, replaced valine (V). Alanine (A), usually found in h-Abs at V_{K}-80, replaced serine (S). Valine (V), always found in h-Abs at V_{K}-83, replaced leucine (L). None of these positions are known to be involved in V_{H}-V_{K} or CDR contacts.

### V_{K} framework regions 4 (V_{K}FR-4)

This short region resembles the human sequences and is acceptable as is.

With a total of only 13 amino acid substitutions made in the V_{H} and V_{K} frameworks of m679 as described above, the new frameworks contain all residues found in h-Abs, except two, namely, leucine at position V_{H}-37, which is retained due to its involvement in the V_{H} and V_{K} contact, and threonine at position V_{H}-110, which is retained because of technical reasons.

### Methods

Eight oligonucleotide PCR primers, which together contain 12 of the 13 mutations described above to convert m679scFv into h679 diabody, were synthesized and used to generate 4 PCR products. The mutant sequences were amplified from the 679scFv-L5 plasmid construct using Taq polymerase. Restriction sites were engineered into the primers to allow ligation of the PCR products while conserving the encoded amino acid sequence. The sequences, coding regions, restriction sites and specific mutations contained on each of the primers are summarized in Table 4. The relative location of the primers and the PCR products are shown schematically in Figure 21. The PCR products were each cloned into the PCR cloning vector pGemT (Promega). Through several rounds of sub-cloning using standard methods, the four PCR sequences were assembled and added to the first 120 nucleotides of 679V_{H}I3Q to generate the h679scFv-L5-pGemT construct. From this construct the V_{H} and V_{K} domains were transferred together into the pET26b expression vector for h679 diabody or individually to make fully humanized bi-specific diabodies. The sub-cloning process is described in detail below.

**Table 4. PCR Primers for humanization of 679scFv-L5**

| Primer | Base Pairs | Restriction Site | Mutations | Sequence |
|---|---|---|---|---|
| A-Left | 121 - 150 | Xma I | V_{H}-E42G | 5'GCTTCCCGGAAAGGGGCTGGA GTGGGTCGCAACC3' |
| | | | V_{H}-R44G | |
| A-Right | 212 - 247 | Pst I | V_{H}-N77S | 5'CGATCTGCAGATATAGGCTGTT |
| | | | | CTTGGCATTGTCTCTGG3' |
| B-Left | 241 - 284 | Pst I | V_{H}-S84A | 5'CTGCAGATGAACAGTCTAAGG GCTGAGGACACGGCCTTGTATTA3' |
| | | | V_{H}-A85E | |
| B-Right | 365 - 421 | Xma I | V_{K}-S5T | 5'CCCCGGGTGACACAGCCAGGGAG GATGGAGATTGTGTCATCACAATGT CGGATCCGC3' |
| C-Left | 414 - 455 | Xma I | V_{K}-K18R | 5'ACCCGGGGAGAGGGTCACTCTGA CCTGCAAATCCAGTCAGAG3' |
| | | | V_{K}-M21L | |
| C-Right | 565 - 595 | Bsp EI | V_{K}-T63S | 5'TTCCGGATCCACTGCCTGAGAAG CGATCAGGGACCCCAGA3' |
| D-Left | 588 - 659 | Bsp EI | V_{K}-V78L | 5'ATCCGGAACAGATTTCACTCTCAC CATCAACAGTCTGCAGGCTGAAGAC GTGGCAGTTTATTACTGCACTCA3' |
| | | | V_{K}-S80A | |
| | | | V_{K}-L83V | |
| D-Right | 687 -717 | Xho I | None | 5'ATCCTCGAGCCGTTTCAGCTCCAG CTTGGT3' |

### Construct A. 1-247 with 3 V_{H} mutations

A plasmid clone containing the 679V_{H}-I3Q mutation (679V_{H}I3Q-pGemT) was digested with the restriction enzymes BspEI (base pair 121) and PstI (in pGemT vector 3' of the insert), leaving the first 121 base pairs of 679V_{H}I3Q with the vector. This vector fragment was ligated with PCR product A that was digested with XmaI (5' end) and Pst I (3' end) to generate construct A. It is important to note that the BspEI-XmaI ligation destroys both sites as each of these restriction enzymes was used in subsequent steps.

### Construct B. 1-415 with 2 additional V_{H} and 1 V_{K} mutations

PCR product B was cloned into pGem T and screened for clones in the T7 orientation. The B fragment was excised from the pGemT clone with PstI and ligated into the PstI site of construct A. Clones were screened for proper insert orientation for construct B.

### Construct C. 1-589 with 3 additional V_{K} mutations

PCR product C was cloned into pGem T and screened for clones in the T7 orientation. The C fragment was excised from the pGemT clone with XmaI and NdeI (vector site) and then ligated into construct B that was digested with the same enzymes.

### Construct D. Humanized 679scFv in pGemT

PCR product D was cloned into pGem T and screened for clones in the T7 orientation. The D fragment was excised from the pGemT clone with BspEI and NdeI and then ligated into construct C that was digested with the same enzymes.

### H679scFv-L5 construction and production of h679 diabody

The h679scFv-L5 sequence was excised from the pGemT construct with NcoI and XhoI and ligated into similarly digested pET26b vector. This construct was used to transform BL21(P-LysS) *E. coli.* Culture conditions, induction, and purification were carried out similar to those described for the m679 diabody in Example 2. Expression levels in the soluble fractions were estimated by BIAcore analysis using a HSG coupled sensor chip. The expression level of h679 diabody was 50 µg/L as compared to 1 ug/L for the wild type m679 diabody or 10 ug/L for m679I3Q diabody. The h679 diabody displayed comparable binding properties to the m679I3Q diabody with BIAcore analysis.

### Example 9 - BS1.5H

Using the methods described in Example 6, the h679V_{H} and h679V_{K} domains were incorporated into the pET-ER vector with the V_{H} and V_{K} of hMN14 to make the fully humanized BS1.5H bispecific diabody construct. The di-cistronic expression vector was constructed through a series of sub-cloning procedures that are outlined in Figure 9. Initially, the V_{K} sequences of h679-scFv-L5 and hMN14-scFv-L5 were exchanged by excision with BamHI and XhoI to generate two intermediate constructs in pET26b. A DNA fragment containing the sequence h679V_{H}-L5-hMN14V_{K}, excised from a pET26b construct with NcoI and XhoI, was ligated into the same restriction sites in pET-ER vector to generate an intermediate clone (h679V_{H}-L5-hMN14V_{K}- pET-ER). A 900bp DNA fragment, which includes a ribosomal binding site in addition to the coding sequence for polypeptide 2 (below), was excised from hMN14V_{H}-L5-h679V_{K}- pET26b with XbaI and BlpI. This fragment was ligated into the SpeI and BlpI restriction sites of h679V_{H}-L5-hMN14V_{K}-pET-ER to create the final bispecific expression construct, BS1.5H. The di-cistronic expression cassette codes for two polypeptides that are arranged as follows:
**Polypeptide 1** Pel B; h679V_{H}; GGGGS linker; hMN14V_{K}; 6 His
**Polypeptide 2** Pel B; hMN14V_{H}; GGGGS linker; h679V_{K}; 6His

When this cassette is expressed in *E. coli,* some of the polypeptides fold and spontaneously form soluble bispecific diabodies.

The BS1.5H construct was used to transform *E. coli* (BL21-pLysS) cells. The recombinant BS1.5H protein was expressed and purified as described in Example 6. The level of soluble protein expression was 0.55 mg/L, about 10% higher than BS1.5. Size exclusion HPLC analysis of the purified BS1.5H yielded a single protein peak at 10.16 minutes (Fig 22). Comparatively, BS2 had a retention time of 10.04 minutes under identical conditions, indicating that BS1.5H polypeptides exclusively form diabodies. The bispecific (CEA/HSG) binding properties of BS1.5H were confirmed by BIAcore analysis (Fig 23). BS1.5H was pre-bound to a HSG-coupled sensor chip before injection of WI2 (hMN14-specific anti-idiotype MAb). As shown in Figure 23, injection of 60 ng of BS1.5H gave a relative response of 660 RU. Subsequent injection of 1 µg of WI2 increased the signal by 760 RU. Injection of WI2 following pre-binding with 679 F(ab')₂ or without pre-binding yielded a negligible response. These data demonstrate that BS1.5H has the capability of binding HSG and CEA simultaneously. BS1.5H differs from BS1.5 by the humanization of the 679 moiety, which was accomplished by substitutions of 13 amino acid residues. To determine if the HSG binding affinity was affected by these changes, BIAcore binding curves for HSG binding of BS1.5H were compared with those of BS1.5 and BS2. As exemplified in Figure 24, the off rates for BS1.5H were very similar to those of BS1.5 and not BS2, which has lower HSG binding affinity. This was consistently the case over a range of analyte concentrations, demonstrating that the HSG binding affinity was largely unaffected by the humanization.

### Example 10 - Linking Moiety / Hapten Conjugate for Carrier Molecule

The therapeutic agents may take the following form:
(Drug)ₘ-Linking Moiety-(Hapten)ₙ

The therapeutic agent comprises at least two haptens which are covalently linked via a peptidic, proteinaceous or non-proteinaceous moiety. Non-limiting examples of haptens are fluorescein isothiocyanate, vitamin B-12, DTPA (diethylenetriaminepentaacetic acid) and DOTA (1,4,7,10-tetraazacyclododecanetetraacetic acid) residues.

This example relates to the preparation of carboxylesterase-DTPA conjugate. Two vials of rabbit liver carboxylesterase (about 8.5 mg protein content/vial) were reconstituted with 2.3 mL of 50 mM potassium phosphate buffer pH 7.5, and the solution was made 4.2 mM in DTPA using 0.1 mL of a 0.1 M stock solution of DTPA pH 6.7. The pH of the resultant solution was adjusted to be in the 7.7-7.8 range, and then reacted with 10 mg of cyclic DTPA dianhydride. After 1 h of stirring at the room temperature, the reaction mixture was passed through two successive SEC columns equilibrated in 0.1 M sodium phosphate pH 7.3. The eluate was further purified by preparative HPLC on a TSK G3000SW column using 0.2 M sodium phosphate pH 6.8, at 4 mL/min flow, as the eluent. The purified conjugate was made 0.1 M in sodium phosphate pH 6.8, and concentrated. The DTPA-to-carboxylesterase molar substitution ratio, determined by a metal-binding assay, was in the range of 2.95-to-1 to 4.43-to-1.

## Claims

1. A multivalent, multi-specific binding protein comprising a histamine-succinyl-glycyl (HSG) binding site having an affinity towards molecules containing a HSG moiety and a carcinoembryonic antigen (CEA) binding site having an affinity towards CEA, wherein said HSG antigen binding site comprises a first and second polypeptide segments that must associate with each other to form said HSG antigen binding site, wherein
said first polypeptide segment comprises a heavy chain variable region comprising an amino acid sequence selected from the group consisting of 
EVILVESGGDLVKPGGSLKLSCAASGFTFSIYTMSWLRQTPGKGLEWVATLSGDGDD
IYYPDSVKGRFTISRDNAKNSLYLQMNSLRAEDTALYYCARVRLGDWDFDVWGQGT
TVSVSS (VH 679) and
 
EVQLVESGGDLVKPGGSLKLSCAASGFTFSIYTMSWLRQTPGKGLEWVATLSGDGD
DIYYPDSVKGRFTISRDNAKNSLYLQMNSLRAEDTALYYCARVRLGDWDFDVWGQ
GTTVSVSS (VH 679 I3Q),
 and said second polypeptide segment comprises a light chain variable region comprising an amino acid sequence selected from the group consisting of

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLCTFGAGTKLELKR
(VK 679) and

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLSTFGAGTKLELKR
(VK 679 C101S).

2. The multivalent, multi-specific binding protein according to claim 1, wherein said first polypeptide segment comprises a heavy chain variable region comprising an amino acid sequence of 
EVILVESGGDLVKPGGSLKLSCAASGFTFSIYTMSWLRQTPGKGLEWVATLSGDGDD
IYYPDSVKGRFTISRDNAKNSLYLQMNSLRAEDTALYYCARVRLGDWDFDVWGQGT
TVSVSS (VH 679 I3Q),
 and said second polypeptide segment comprises a light chain variable region comprising an amino acid sequence of

IVMTQSPSSLAVSPGERVTLTCKSSQSLFNSRTRKNYLGWYQQKPGQSPKLLIYWAST
RESGVPDRFSGSGSGTDFTLTINSLQAEDVAVYYCTQVYYLCTFGAGTKLELKR (VK
679).

3. The multivalent, multi-specific binding protein according to claim 1, wherein said first polypeptide segment comprises a heavy chain variable region comprising an amino acid sequence of 
EVQLVESGGDLVKPGGSLKLSCAASGFTFSIYTMSWLRQTPGKGLEWVATLSGDGD
DIYYPDSVKGRFTISRDNAKNSLYLQMNSLRAEDTALYYCARVRLGDWDFDVWGQ
GTTVSVSS (VH 679 I3Q),
 and said second polypeptide segment comprises a light chain variable region comprising an amino acid sequence of

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLSTFGAGTKLELKR
(VK 679 C101S).

4. The binding protein of claim 1, wherein said CEA antigen binding site comprises a third and fourth polypeptide segments that must associate with each other to form said CEA antigen binding site.

5. The binding protein of claim 4, wherein said third polypeptide segment comprises a VH polypeptide of hMN14 MAb of Fig. 29, and said fourth polypeptide segment comprises a VK polypeptide of hMN14 MAb of Fig. 29.

6. The binding protein of claim 1, wherein said first and third polypeptide segments are connected by a first linker and said second and fourth polypeptide segments are connected by a second linker.

7. The binding protein of claim 6, wherein said first linker and said second linker each comprise five or less amino acid residues.

8. The binding protein of claim 1, wherein said binding protein is a diabody.

9. The binding protein of claim 8, wherein said diabody is selected from the group consisting of a diabody comprising amino acid sequences shown in Fig. 30 and Fig. 31, a diabody comprising amino acid sequences shown in Fig. 32 and Fig. 33, a diabody comprising amino acid sequences shown in Fig. 34 and Fig. 35, and a diabody comprising amino acid sequences shown in Fig. 37 and 38.

10. The binding protein of claim 6, wherein said first, second, third and fourth polypeptide segments are each encoded by a first, second, third and fourth cDNA, respectively.

11. The binding protein of claim 10, wherein said first cDNA comprises nucleotide sequences shown in Fig. 30 and Fig. 31, wherein said second cDNA comprises nucleotide sequences shown in Fig. 32 and Fig. 33, wherein said third cDNA comprises nucleotide sequences shown in Fig. 34 and 35, and wherein said fourth cDNA comprises nucleotide sequences shown in Fig. 37 and 38.

12. The binding protein of claim 10, wherein said first and third cDNAs are contained on a single nucleic acid molecule, or wherein said second and fourth cDNAs are contained on a single nucleic acid molecule, or wherein said first, second, third and fourth cDNAs are on a single expression cassette, wherein preferably said expression cassette is contained in a plasmid.

13. A host cell comprising a plasmid as defined in claim 12.

14. A method of producing a binding protein, comprising culturing the host cell of claim 13 in a suitable medium, and separating said binding protein from said media.

15. The binding protein of claim 1 for use in treatment of a disease, wherein the treatment comprises delivering a therapeutic agent, or a combination of the therapeutic agent and a diagnostic agent to a target, comprising:
a) administering the binding protein to a subject;
b) waiting a sufficient amount of time for an amount of the non-bound binding protein to clear the subject's blood stream; and
c) administering to said subject a carrier molecule comprising the therapeutic agent or a combination of the therapeutic agent and a diagnostic agent, that binds to a binding site of the binding protein.

16. The binding protein of claim 1 for use in diagnosis of disease, wherein the diagnosis comprises delivering a diagnostic agent or a combination of the diagnostic agent and a therapeutic agent to a target, comprising:
a) administering the binding protein to a subject;
b) waiting a sufficient amount of time for an amount of the non-bound binding protein to clear the subject's blood stream; and
c) administering to said subject a carrier molecule comprising the diagnostic agent or a combination of the diagnostic agent and a therapeutic agent, that binds to a binding site of the binding protein.

17. The binding protein for use of claims 15 and 16, wherein said carrier molecule binds to more than one binding site of the binding protein.

18. The binding protein for use of claim 15, wherein said therapeutic agent is selected from the group comprising, drugs, toxins, cytokines, hormones, growth factors, conjugates, and radionuclides.

19. The use of a binding protein of claim 1 in the preparation of a medicament for detecting or treating a human disorder, wherein the binding protein of claim 1 is administered to a subject; a sufficient amount of time is waited for an amount of the non-bound binding protein to clear the subject's blood stream; and a carrier molecule comprising a diagnostic agent, a therapeutic agent, or a combination thereof, that binds to a binding site of the binding protein is administered to the subject.

20. The use of claim 19, wherein said human disorder is selected from the group comprising cancer, autoimmune diseases, infectious diseases, cardiovascular diseases, and inflammatory diseases.

## Patentansprüche

1. Multivalentes, multi-spezifisches Bindungsprotein umfassend eine Bindungsstelle für Histamin-Succinyl-Glycyl (HSG) mit einer Affinität für Moleküle enthaltend einen HSG-Anteil und eine Bindungsstelle für karzinoembryonisches Antigen (CEA) mit einer Affinität für CEA, wobei die HSG-Antigen-Bindungsstelle ein erstes und zweites Polypeptidsegment umfasst, die miteinander assoziieren müssen, um die HSG-Antigen-Bindungsstelle auszubilden, wobei
das erste Polypeptidsegment eine variable Region einer schweren Kette umfasst, die eine Aminosäuresequenz umfasst, die ausgewählt ist aus der Gruppe bestehend aus 
EVILVESGGDLVKPGGSLKLSCAASGFTFSIYTMSWLRQTPGKGLEWVATLSGDGDD
IYYPDSVKGRFTISRDNAKNSLYLQMNSLRAEDTALYYCARVRLGDWDFDVWGQGT
TVSVSS (VH 679) und
 
EVQLVESGGDLVKPGGSLKLSCAASGFTFSIYTMSWLRQTPGKGLEWVATLSGDGD
DIYYPDSVKGRFTISRDNAKNSLYLQMNSLRAEDTALYYCARVRLGDWDFDVWGQ
GTTVSVSS (VH 679 I3Q),
 und das zweite Polypeptidsegment eine variable Region einer leichten Kette umfasst, die eine Aminosäuresequenz umfasst, die ausgewählt ist aus der Gruppe bestehend aus

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLCTFGAGTKLELKR
(VK 679) und

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLSTFGAGTKLELKR
(VK 679 C101S).

2. Multivalentes, multi-spezifisches Bindungsprotein nach Anspruch 1, wobei das erste Polypeptidsegment eine variable Region einer schweren Kette umfasst, die eine Aminosäuresequenz umfasst aus 
EVILVESGGDLVKPGGSLKLSCAASGFTFSIYTMSWLRQTPGKGLEWVATLSGDGDD
IYYPDSVKGRFTISRDNAKNSLYLQMNSLRAEDTALYYCARVRLGDWDFDVWGQGT
TVSVSS (VH 679 I3Q),
 und das zweite Polypeptidsegment eine variable Region einer leichten Kette umfasst, die eine Aminosäuresequenz umfasst aus

IVMTQSPSSLAVSPGERVTLTCKSSQSLFNSRTRKNYLGWYQQKPGQSPKLLIYWAST
RESGVPDRFSGSGSGTDFTLTINSLQAEDVAVYYCTQVYYLCTFGAGTKLELKR (VK
679).

3. Multivalentes, multi-spezifisches Bindungsprotein nach Anspruch 1, wobei das erste Polypeptidsegment eine variable Region einer schweren Kette umfasst, die eine Aminosäuresequenz umfasst aus 
EVQLVESGGDLVKPGGSLKLSCAASGFTFSIYTMSWLRQTPGKGLEWVATLSGDGD
DIYYPDSVKGRFTISRDNAKNSLYLQMNSLRAEDTALYYCARVRLGDWDFDVWGQ
GTTVSVSS (VH 679 I3Q),
 und das zweite Polypeptidsegment eine variable Region einer leichten Kette umfasst, die eine Aminosäuresequenz umfasst aus

DIVMTQSPSSLAVSPGERVTLTCKSSQSLFNSRTRKNYLGWYQQKPGQSPKLLIYWAS
TRESGVPDRFSGSGSGTDFTLTINSLQAEDVAVYYCTQVYYLSTFGAGTKLELKR
(VK 679 C101S).

4. Bindungsprotein nach Anspruch 1, wobei die CEA-Antigen-Bindungsstelle ein drittes und viertes Polypeptidsegment umfasst, die miteinander assoziieren müssen, um die CEA-Antigen-Bindungsstelle auszubilden.

5. Bindungsprotein nach Anspruch 4, wobei das dritte Polypeptidsegment ein VH-Polypeptid von MAb hMN14 von Fig. 29 und das vierte Polypeptidsegment ein VK-Polypeptid von MAb hMN 14 von Fig. 29 umfasst.

6. Bindungsprotein nach Anspruch 1, wobei das erste und dritte Polypeptidsegment durch einen ersten Linker miteinander verbunden sind und das zweite und vierte Polypeptidsegment durch einen zweiten Linker miteinander verbunden sind.

7. Bindungsprotein nach Anspruch 6, wobei der erste Linker und der zweite Linker jeweils fünf oder weniger Aminosäurereste umfassen.

8. Bindungsprotein nach Anspruch 1, wobei das Bindungsprotein ein Diabody ist.

9. Bindungsprotein nach Anspruch 8, wobei der Diabody ausgewählt ist aus der Gruppe bestehend aus einem Diabody umfassend Aminosäuresequenzen, wie in Fig. 30 und Fig. 31 gezeigt, einen Diabody umfassend Aminosäuresequenzen, wie in Fig. 32 und Fig. 33 gezeigt, einen Diabody umfassend Aminosäuresequenzen, wie in Fig. 34 und Fig. 35 gezeigt, und einen Diabody umfassend Aminosäuresequenzen, wie in Fig. 37 und 38 gezeigt.

10. Bindungsprotein nach Anspruch 6, wobei das erste, zweite, dritte und vierte Polypeptidsegment jeweils durch eine erste, zweite, dritte beziehungsweise vierte cDNA codiert ist.

11. Bindungsprotein nach Anspruch 10, wobei die erste cDNA Nukleotidsequenzen umfasst, wie in Fig. 30 und Fig. 31 gezeigt, wobei die zweite cDNA Nukleotidsequenzen umfasst, wie in Fig. 32 und Fig. 33 gezeigt, wobei die dritte cDNA Nukleotidsequenzen umfasst, wie in Fig. 34 und 35 gezeigt, und wobei die vierte cDNA Nukleotidsequenzen umfasst, wie in Fig. 37 und 38 gezeigt.

12. Bindungsprotein nach Anspruch 10, wobei die erste und dritte cDNA auf einem einzelnen Nukleinsäuremolekül enthalten sind, oder wobei die zweite und vierte cDNA auf einem einzelnen Nukleinsäuremolekül enthalten sind, oder wobei sich die erste, zweite, dritte und vierte cDNA auf einer einzelnen Expressionskassette befinden, wobei bevorzugterweise die Expressionskassette in einem Plasmid enthalten ist.

13. Wirtszelle umfassend ein Plasmid, wie in Anspruch 12 definiert.

14. Verfahren zum Herstellen eines Bindungsproteins, umfassend Kultivieren der Wirtszelle von Anspruch 13 in einem geeigneten Medium und Abtrennen des Bindungsproteins von dem Medium.

15. Bindungsprotein nach Anspruch 1 zur Verwendung bei der Behandlung einer Erkrankung, wobei die Behandlung umfasst Abgabe eines therapeutischen Agens oder einer Kombination aus dem therapeutischen Agens und einem diagnostischen Agens an ein Ziel, umfassend:
a) Verabreichen des Bindungsproteins an ein Lebewesen;
b) Abwarten einer ausreichenden Zeitspanne, damit eine Menge des nicht-gebundenen Bindungsproteins aus dem Blutstrom des Lebewesens geklärt wird; und
c) Verabreichen an das Lebewesen eines Trägermoleküls umfassend das therapeutische Agens oder eine Kombination aus dem therapeutischen Agens und einem diagnostischen Agens, das an eine Bindungsstelle des Bindungsproteins bindet.

16. Bindungsprotein nach Anspruch 1 zur Verwendung bei der Diagnose einer Erkrankung, wobei die Diagnose umfasst Abgabe eines diagnostischen Agens oder einer Kombination aus dem diagnostischen Agens und einem therapeutischen Agens an ein Ziel, umfassend:
a) Verabreichen des Bindungsproteins an ein Lebewesen;
b) Abwarten einer ausreichenden Zeitspanne, damit eine Menge des nicht-gebundenen Bindungsproteins aus dem Blutstrom des Lebewesens geklärt wird; und
c) Verabreichen an das Lebewesen eines Trägermoleküls umfassend das diagnostische Agens oder eine Kombination aus dem diagnostischen Agens und einem therapeutischen Agens, das an eine Bindungsstelle des Bindungsproteins bindet.

17. Bindungsprotein zur Verwendung nach Anspruch 15 und 16, wobei das Trägermolekül an mehr als eine Bindungsstelle des Bindungsproteins bindet.

18. Bindungsprotein zur Verwendung nach Anspruch 15, wobei das therapeutische Agens ausgewählt ist aus der Gruppe umfassend Wirkstoffe, Toxine, Zytokine, Hormone, Wachstumsfaktoren, Konjugate und Radionuklide.

19. Verwendung eines Bindungsproteins nach Anspruch 1 bei der Herstellung eines Medikaments zum Nachweis oder Behandeln einer menschlichen Erkrankung, wobei das Bindungsprotein nach Anspruch 1 einem Lebewesen verabreicht wird, eine ausreichende Zeitspanne abgewartet wird, damit eine Menge des nicht-gebundenen Bindungsproteins aus dem Blutstrom des Lebewesens geklärt wird; und ein Trägermolekül umfassend ein diagnostisches Agens, ein therapeutisches Agens oder eine Kombination davon, das an eine Bindungsstelle des Bindungsproteins bindet, dem Lebewesen verabreicht wird.

20. Verwendung nach Anspruch 19, wobei die menschliche Erkrankung ausgewählt ist aus der Gruppe umfassend Krebs, Autoimmunerkrankung, Infektionskrankheiten, kardiovaskuläre Erkrankungen und Entzündungskrankheiten.

## Revendications

1. Protéine de liaison multivalente, multi-spécifique comprenant un site de liaison histamine-succinyl-glycyle (HSG) ayant une affinité pour des molécules contenant un fragment HSG et un site de liaison d'antigène carcino-embryonnaire (CEA) ayant une affinité pour CEA, où ledit site de liaison d'antigène HSG comprend des premier et deuxième segments de polypeptide qui doivent s'associer l'un avec l'autre pour former ledit site de liaison d'antigène HSG, dans laquelle ledit premier segment de polypeptide comprend une région variable de chaîne lourde comprenant une séquence d'acides aminés choisie dans le groupe constitué de 
EVILVESGGDLVKPGGSLKLSCAASGFTFSIYTMSWLRQTPGKGLEWVATLSGDGDDIYYPDSVK
GRFTISRDNAKNSLYLQMNSLRAEDTALYYCARVRLGDWDFDVWGQGTTVSVSS (VH 679) et
 
EVQLVESGGDLVKPGGSLKLSCAASGFTFSIYTMSWLRQTPGKGLEWVATLSGDGDDIYYPDSV
KGRFTISRDNAKNSLYLQMNSLRAEDTALYYCARVRLGDWDFDVWGQGTTVSVSS (VH 679
I3Q),
 et ledit deuxième segment de polypeptide comprend une région variable de chaîne légère comprenant une séquence d'acides aminés choisie dans le groupe constitué de

DIVMTQSPSSLAVSPGERVTLTCKSSQSLFNSRTRKNYLGWYQQKPGQSPKLLIYWASTRESGV
PDRFSGSGSGTDFTLTINSLQAEDVAVYYCTQVYYLCTFGAGTKLELKR (VK 679) et

DIVMTQSPSSLAVSPGERVTLTCKSSQSLFNSRTRKNYLGWYQQKPGQSPKLLIYWASTRESGV
PDRFSGSGSGTDFTLTINSLQAEDVAVYYCTQVYYLSTFGAGTKLELKR
(VK 679 C101S).

2. Protéine de liaison multivalente, multi-spécifique selon la revendication 1, dans laquelle ledit premier segment de polypeptide comprend une région variable de chaîne lourde comprenant une séquence d'acides aminés de 
EVILVESGGDLVKPGGSLKLSCAASGFTFSIYTMSWLRQTPGKGLEWVATLSGDGDDIYYPDSVK
GRFTISRDNAKNSLYLQMNSLRAEDTALYYCARVRLGDWDFDVWGQGTTVSVSS (VH 679
I3Q),
 et ledit deuxième segment de polypeptide comprend une région variable de chaîne légère comprenant une séquence d'acides aminés de

IVMTQSPSSLAVSPGERVTLTCKSSQSLFNSRTRKNYLGWYQQKPGQSPKLLIYWASTRESGVP
DRFSGSGSGTDFTLTINSLQAEDVAVYYCTQVYYLCTFGAGTKLELKR (VK 679).

3. Protéine de liaison multivalente, multi-spécifique selon la revendication 1, dans laquelle ledit premier segment de polypeptide comprend une région variable de chaîne lourde comprenant une séquence d'acides aminés de 
EVQLVESGGDLVKPGGSLKLSCAASGFTFSIYTMSWLRQTPGKGLEWVATLSGDGDDIYYPDSV
KGRFTISRDNAKNSLYLQMNSLRAEDTALYYCARVRLGDWDFDVWGQGTTVSVSS (VH 679
I3Q),
 et ledit deuxième segment de polypeptide comprend une région variable de chaîne légère comprenant une séquence d'acides aminés de

DIVMTQSPSSLAVSPGERVTLTCKSSQSLFNSRTRKNYLGWYQQKPGQSPKLLIYWASTRESGV
PDRFSGSGSGTDFTLTINSLQAEDVAVYYCTQVYYLSTFGAGTKLELKR (VK 679 C101S).

4. Protéine de liaison de la revendication 1, dans laquelle ledit site de liaison d'antigène CEA comprend des troisième et quatrième segments de polypeptide qui doivent s'associer l'un à l'autre pour former ledit site de liaison d'antigène CEA.

5. Protéine de liaison de la revendication 4, dans laquelle ledit troisième segment de polypeptide comprend un polypeptide VH du MAb hMN14 de la figure 29, et ledit quatrième segment de polypeptide comprend un polypeptide VK du MAb hMN14 de la figure 29.

6. Protéine de liaison de la revendication 1, dans laquelle lesdits premier et troisième segments de polypeptide sont reliés par un premier lieur et lesdits deuxième et quatrième segments de polypeptide sont reliés par un deuxième lieur.

7. Protéine de liaison de la revendication 6, dans laquelle ledit premier lieur et ledit deuxième lieur comprennent chacun cinq résidus d'acide aminé ou moins.

8. Protéine de liaison de la revendication 1, dans laquelle ladite protéine de liaison est un dianticorps (diabody).

9. Protéine de liaison de la revendication 8, dans laquelle ledit dianticorps est choisi dans le groupe constitué d'un dianticorps comprenant les séquences d'acides aminés décrites sur la figure 30 et la figure 31, un dianticorps comprenant les séquences d'acides aminés décrites sur la figure 32 et la figure 33, un dianticorps comprenant les séquences d'acides aminés décrites sur la figure 34 et la figure 35, et un dianticorps comprenant les séquences d'acides aminés décrites sur la figure 37 et 38.

10. Protéine de liaison de la revendication 6, dans laquelle lesdits premier, deuxième, troisième et quatrième segments de polypeptide sont chacun codés par un premier, un deuxième, un troisième et un quatrième ADNc, respectivement.

11. Protéine de liaison de la revendication 10, où ledit premier ADNc comprend les séquences nucléotidiques décrites sur la figure 30 et la figure 31, où ledit deuxième ADNc comprend les séquences nucléotidiques décrites sur la figure 32 et la figure 33, où ledit troisième ADNc comprend les séquences nucléotidiques décrites sur la figure 34 et 35, et où ledit quatrième ADNc comprend les séquences nucléotidiques décrites sur les figures 37 et 38.

12. Protéine de liaison de la revendication 10, où lesdits premier et troisième ADNc sont contenus sur une molécule d'acide nucléique unique, ou lesdits deuxième et quatrième ADNc sont contenus sur une molécule d'acide nucléique unique, ou lesdits premier, deuxième, troisième et quatrième ADNc sont sur une cassette d'expression unique, où, de préférence, ladite cassette d'expression est contenue dans un plasmide.

13. Cellule hôte comprenant un plasmide tel que défini dans la revendication 12.

14. Procédé de production d'une protéine de liaison, comprenant la culture de la cellule hôte de la revendication 13 dans un milieu adapté, et la séparation de ladite protéine de liaison dudit milieu.

15. Protéine de liaison de la revendication 1 pour utilisation dans le traitement d'une maladie, où le traitement comprend l'administration d'un agent thérapeutique, ou d'une combinaison de l'agent thérapeutique et d'un agent diagnostique à une cible, comprenant :
a) l'administration de la protéine de liaison à un sujet ;
b) l'attente d'une durée suffisante pour qu'une quantité de la protéine de liaison non liée soit éliminée de la circulation sanguine du sujet ; et
c) l'administration audit sujet d'une molécule de support comprenant l'agent thérapeutique ou une combinaison de l'agent thérapeutique et d'un agent diagnostique, qui se lie à un site de liaison de la protéine de liaison.

16. Protéine de liaison de la revendication 1 pour utilisation dans le diagnostic d'une maladie, où le diagnostic comprend l'administration d'un agent diagnostique ou d'une combinaison de l'agent diagnostique et d'un agent thérapeutique à une cible, comprenant :
a) l'administration de la protéine de liaison à un sujet ;
b) l'attente d'une durée suffisante pour qu'une quantité de la protéine de liaison non liée soit éliminée de la circulation sanguine du sujet ; et
c) l'administration audit sujet d'une molécule de support comprenant l'agent diagnostique ou une combinaison de l'agent diagnostique et d'un agent thérapeutique, qui se lie à un site de liaison de la protéine de liaison.

17. Protéine de liaison pour utilisation des revendications 15 et 16, **caractérisée en ce que** ladite molécule de support se lie à plus d'un site de liaison de la protéine de liaison.

18. Protéine de liaison pour utilisation de la revendication 15, où ledit agent thérapeutique est choisi dans le groupe comprenant des médicaments, des toxines, des cytokines, des hormones, des facteurs de croissance, des conjugués et des radionucléides.

19. Utilisation d'une protéine de liaison de la revendication 1 dans la préparation d'un médicament pour détecter ou traiter un trouble humain, dans laquelle la protéine de liaison de la revendication 1 est administrée à un sujet ; une durée suffisante est attendue pour qu'une quantité de la protéine de liaison non liée soit éliminée de la circulation sanguine du sujet ; et une molécule de support comprenant un agent diagnostique, un agent thérapeutique, ou une combinaison de ceux-ci, qui se lie à un site de liaison de la protéine de liaison est administrée au sujet.

20. Utilisation de la revendication 19, dans laquelle ledit trouble humain est choisi dans le groupe comprenant un cancer, des maladies auto-immunes, des maladies infectieuses, des maladies cardiovasculaires et des maladies inflammatoires.
